# EUROPÄISCHE PATENTSCHRIFT

(11) **EP 3 180 383 B1**
(45) Veröffentlichungstag und Bekanntmachung des Hinweises auf die Patenterteilung: **10.10.2018**
(21) Anmeldenummer: 15750382.2
(22) Anmeldetag: 07.08.2015
(51) Int. Cl.: C08G 59/50, C07C 211/27, C09D 163/00, C08L 63/00

(54) **AMIN FÜR EMISSIONSARME EPOXIDHARZ-ZUSAMMENSETZUNGEN**
AMINE FOR LOW-EMISSION EPOXY RESIN COMPOSITIONS
AMINES À FAIBLE D'ÉMISSION DES COMPOSITIONS DE RÉSINE ÉPOXY

(30) Priorität: 13.08.2014 EP 14180869
(43) Veröffentlichungstag der Anmeldung: 21.06.2017
(73) Patentinhaber: Sika Technology AG, 6340 Baar (CH)
(72) Erfinder: KASEMI, Edis, CH-8046 Zürich (CH); KRAMER, Andreas, CH-8006 Zürich (CH); STADELMANN, Ursula, CH-8046 Zürich (CH); BURCKHARDT, Urs, CH-8049 Zürich (CH)
(74) Vertreter: Sika Patent Attorneys
(86) Internationale Anmeldenummer: PCT/EP2015/068302
(87) Internationale Veröffentlichungsnummer: WO 2016/023839

(56) Entgegenhaltungen:
- EP-A1- 2 752 403
- US-A1- 2014 107 313

## Beschreibung

### Technisches Gebiet

Die Erfindung betrifft das Gebiet der Amine, Härter für Epoxidharze, Epoxidharz-Zusammensetzungen, sowie deren Verwendung, insbesondere als Beschichtung, Belag oder Anstrich.

### Stand der Technik

Für Beschichtungszwecke geeignete Epoxidharz-Zusammensetzungen sollen eine möglichst niedrige Viskosität aufweisen, damit sie bei Umgebungstemperatur gut verarbeitbar sind. Weiterhin sollen sie möglichst schnell und störungsfrei aushärten, auch bei feucht-kalten Bedingungen, und dabei eine ebenmässige Oberfläche ohne Trübungen, Flecken oder Krater ausbilden. Schliesslich soll eine ausgehärtete Beschichtung eine hohe Härte bei geringer Sprödigkeit besitzen, um mechanischer Beanspruchung möglichst gut zu widerstehen. Für optisch anspruchsvolle Anwendungen, beispielsweise Deckbeläge von Fussböden, soll eine Beschichtung ausserdem einen hohen Glanzgrad und eine möglichst geringe Neigung zum Vergilben unter Lichteinfluss aufweisen.

Aus dem Stand der Technik bekannte Härter für Epoxidharz-Beschichtungen enthalten typischerweise Addukte von Polyaminen mit Epoxiden, insbesondere mit Bisphenol-Flüssigharzen. Solche Addukte ermöglichen eine rasche Aushärtung, sind aber sehr hochviskos, weshalb die Härter zur Einstellung einer handhabbaren Viskosität üblicherweise beträchtliche Anteile an nicht adduktierten Polyaminen und/oder Verdünnern enthalten. Die nicht adduktierten Polyamine sind typischerweise geruchsintensiv und führen zu verstärktem Auftreten von Blushing-Effekten. Als "Blushing-Effekte" werden bei der Aushärtung auftretende Oberflächenmängel wie Trübungen, Flecken, Rauheit und Klebrigkeit bezeichnet, welche durch Salzbildung ("Blushing") von Aminen mit Kohlendioxid (CO₂) aus der Luft verursacht werden und besonders bei hoher Luftfeuchtigkeit und tiefen Temperaturen auftreten. Die Verdünner vermindern typischerweise Blushing-Effekte und verbessern Oberflächenqualität und Sprödigkeit der Beschichtung, werden aber bei der Aushärtung nicht in die Harzmatrix eingebaut und können durch Verdampfungs- oder Diffusionsprozesse freigesetzt werden. Heutzutage werden aber zunehmend emissionsarme Produkte gewünscht, die nach der Aushärtung einen geringen Gehalt an freisetzbaren Substanzen aufweisen. Für emissionsarme Epoxidharz-Zusammensetzungen können Verdünner, wie beispielsweise Benzylalkohol, deshalb nur in geringer Menge oder gar nicht verwendet werden.

Aus US 2014/0107313 und EP 2 752 403 sind Amine bekannt, welche Epoxidharz-Zusammensetzungen gut verdünnen und kaum zu Blushing-Effekten neigen. Hinsichtlich Aushärtungsgeschwindigkeit und/oder Vergilbung der damit erhaltenen Epoxidharz-Zusammensetzungen sind diese Amine aber noch verbesserungsfähig.

### Darstellung der Erfindung

Aufgabe der vorliegenden Erfindung ist es daher, ein geruchsarmes Amin zur Verwendung in Härtern für raumtemperaturhärtende Epoxidharz-Zusammensetzungen zur Verfügung zu stellen, welches eine stark verdünnende Wirkung ausübt und emissionsarme Epoxidharz-Beschichtungen mit guter Verarbeitbarkeit ermöglicht, die auch in der Kälte ausreichend schnell aushärten und Beschichtungen von hoher Härte, guter Oberflächenqualität und geringer Vergilbungsneigung ergeben.

Diese Aufgabe wird mit dem Amin der Formel (I) gelöst, wie in Anspruch 1 beschrieben. Das Amin der Formel (I) ist geruchsarm und übt eine überraschend stark verdünnende Wirkung auf die üblicherweise verwendeten Epoxidharze aus, ohne Blushing-Effekte oder Unverträglichkeiten auszulösen. Es ermöglicht emissionsarme Epoxidharz-Beschichtungen mit ausgezeichneter Verarbeitbarkeit, die überraschend schnell aushärten, auch bei verhältnismässig tiefen Temperaturen, beispielsweise bei 8 °C, wobei diese über eine überraschend hohe Endhärte und eine glänzende, ebenmässige, nichtklebrige Oberfläche ohne Trübungen, Flecken oder Krater verfügen, welche unter Lichteinfluss überraschenderweise praktisch nicht vergilbt.

Das Amin der Formel (I) ist insbesondere verwendbar als Bestandteil von Härtern, welche ansonsten ohne den Einsatz von Verdünnern zu hochviskos für Beschichtungsanwendungen wären. Besonders vorteilhaft verwendbar ist das Amin der Formel (I) zusammen mit weiteren Aminen, insbesondere zusammen mit aminfunktionellen Addukten aus Polyaminen und Epoxiden. Solche Härter sind überraschend niedrigviskos und härten überraschend schnell aus. Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist die Verwendung eines Amins der Formel (I) als Härter für Epoxidharze, 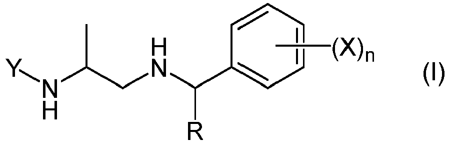 wobei
n für 0 oder 1 oder 2 oder 3 steht,
R für einen Wasserstoff-Rest oder für Methyl oder Phenyl steht,
X für gleiche oder verschiedene Reste ausgewählt aus der Gruppe bestehend aus Alkyl, Alkoxy und Dialkylamino mit jeweils 1 bis 18 Kohlenstoffatomen steht, und
Y für einen Wasserstoff-Rest oder einen Rest der Formel 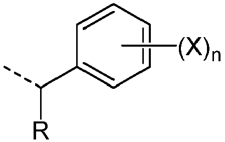 steht.

Mit "Poly" beginnende Substanznamen wie Polyamin, Polyol oder Polyepoxid bezeichnen Substanzen, die formal zwei oder mehr der in ihrem Namen vorkommenden funktionellen Gruppen pro Molekül enthalten.
Als "primäre Aminogruppe" wird eine NH₂-Gruppe bezeichnet, die an einen organischen Rest gebunden ist und als "sekundäre Aminogruppe" wird eine NH-Gruppe bezeichnet, die an zwei organische Reste, welche auch gemeinsam Teil eines Rings sein können, gebunden ist.

Als "Aminwasserstoff" werden die Wasserstoffatome von primären und sekundären Aminogruppen bezeichnet.
Als "Aminwasserstoff-Equivalentgewicht" wird der Gewichtsanteil eines Härters oder eines Amins pro im Härter oder im Amin vorhandenem Aminwasserstoff bezeichnet.
Als "nicht einbaubarer Verdünner" wird eine in einem Epoxidharz lösliche und dessen Viskosität senkende Substanz bezeichnet, welche bei der Aushärtung des Epoxidharzes nicht kovalent in die Harzmatrix eingebaut wird.
Mit dem Begriff "Viskosität" wird im vorliegenden Dokument die dynamische Viskosität oder Scherviskosität bezeichnet, welche durch das Verhältnis zwischen der Schubspannung und der Scherrate (Geschwindigkeitsgefälle) definiert ist und wie in den Ausführungsbeispielen beschrieben bestimmt wird. Eine gestrichelte Linie in den Formeln in diesem Dokument stellt jeweils die Bindung zwischen einem Substituenten und dem zugehörigen Molekülrest dar. Unter "Molekulargewicht" versteht man im vorliegenden Dokument die molare Masse (in Gramm pro Mol) eines Moleküls. Als "mittleres Molekulargewicht" wird das Zahlenmittel Mₙ einer oligomeren oder polymeren Mischung von Molekülen bezeichnet, welches üblicherweise mittels Gelpermeationschromatographie (GPC) gegen Polystyrol als Standard bestimmt wird.
Als "Raumtemperatur" wird eine Temperatur von 23 °C bezeichnet.

Bevorzugt steht n für 0 oder 1 oder 2, besonders bevorzugt für 0 oder 1. Diese Amine ermöglichen besonders niedrigviskose Epoxidharz-Zusammensetzungen.
Ein Amin, bei welchem n für 0 steht, verdünnt besonders gut.
Ein Amin, bei welchem n für 1 steht, ist besonders geruchsarm und kann, je nach Gruppe X, eine besonders schnelle Aushärtung ermöglichen und/oder besonders gut verdünnen.
Am meisten bevorzugt steht n für 0.

Bevorzugt steht X für gleiche oder verschiedene Reste ausgewählt aus der Gruppe bestehend aus Alkyl, Alkoxy und Dialkylamino mit jeweils 1 bis 12, insbesondere 1 bis 4, Kohlenstoffatomen. Besonders bevorzugt steht X für Methyl oder für Methoxy oder für Dimethylamino.

Bevorzugt steht Y für einen Wasserstoff-Rest. Diese Amine ermöglichen besonders niedrigviskose Epoxidharz-Zusammensetzungen mit besonders schneller Aushärtung.

Bevorzugt steht R für einen Wasserstoff-Rest oder für Methyl, insbesondere für einen Wasserstoff-Rest. Diese Amine ermöglichen besonders niedrigviskose Epoxidharz-Zusammensetzungen.

Besonders bevorzugt ist ein Amin der Formel (I), bei welchem Y für einen Wasserstoff-Rest und n für 0 steht. Diese Amine sind besonders einfach zugänglich und ermöglichen besonders niedrigviskose Epoxidharz-Zusammensetzungen, welche sehr schnell aushärten.

Weiterhin besonders bevorzugt ist ein Amin der Formel (I), bei welchem Y für einen Wasserstoff-Rest, n für 1 und X für Methoxy oder für Dimethylamino steht. Diese Amine ermöglichen Epoxidharz-Zusammensetzungen mit besonders schneller Aushärtung. Bevorzugt steht die Methoxygruppe oder die Dimethylaminogruppe in para-Stellung.

Ganz besonders bevorzugte Amine der Formel (I) sind ausgewählt aus der Gruppe bestehend aus N¹-Benzyl-1,2-propandiamin, N¹-(4-lsopropylbenzyl)-1,2-propandiamin, N¹-(4-tert.Butylbenzyl)-1,2-propandiamin, N¹-(4-Methoxybenzyl)-1,2-propandiamin, N¹-(4-(Dimethylamino)benzyl)-1,2-propandiamin, N¹-(1-Phenylethyl)-1,2-propandiamin, N¹-Benzhydryl-1,2-propandiamin, N¹-(1-(4'-Methyl)phenylethyl)-1,2-propandiamin und N¹-(1-(4'-Methoxy)phenylethyl)-1,2-propandiamin.

Davon ganz besonders bevorzugt ist N¹-Benzyl-1,2-propandiamin. Dabei stehen Y und R jeweils für einen Wasserstoff-Rest und n für 0. Dieses Amin ermöglicht geruchs- und emissionsarme Epoxidharz-Zusammensetzungen mit ganz besonders niedriger Viskosität, schneller Härteentwicklung bzw. Aushärtung und überraschend hoher Härte, welche auch bei feucht-kalten Bedingungen kaum Blushing-bedingte Oberflächenstörungen zeigen und überraschenderweise praktisch nicht vergilben.
Davon ganz besonders bevorzugt ist weiterhin N¹-(4-Methoxybenzyl)-1,2-propandiamin. Dabei stehen Y und R jeweils für einen Wasserstoff-Rest, n für 1 und X für einen Methoxy-Rest in 4-Stellung. Dieses Amin ermöglicht geruchs- und emissionsarme Epoxidharz-Zusammensetzungen mit sehr niedriger Viskosität, besonders schneller Härteentwicklung bzw. Aushärtung und hoher Härte, welche auch bei feucht-kalten Bedingungen kaum Blushing-bedingte Oberflächenstörungen zeigen und überraschenderweise praktisch nicht vergilben.
Davon ganz besonders bevorzugt ist weiterhin N¹-(4-(Dimethylamino)benzyl)-1,2-propandiamin. Dabei stehen Y und R jeweils für einen Wasserstoff-Rest, n für 1 und X für einen Dimethylamino-Rest in 4-Stellung. Dieses Amin ermöglicht geruchs- und emissionsarme Epoxidharz-Zusammensetzungen mit sehr niedriger Viskosität, ganz besonders schneller Härteentwicklung bzw. Aushärtung und besonders hoher Härte, welche auch bei feucht-kalten Bedingungen kaum Blushing-bedingte Oberflächenstörungen zeigen und überraschenderweise praktisch nicht vergilben.

Das Amin der Formel (I) wird bevorzugt erhalten aus der reduktiven Alkylierung von 1,2-Propylendiamin mit mindestens einem Aldehyd oder Keton der Formel (II) und Wasserstoff. Daraus erhaltene Reaktionsprodukte weisen einen hohen Gehalt an Aminen der Formel (I) auf und sind besonders geeignet zur Verwendung als Härter für Epoxidharze.

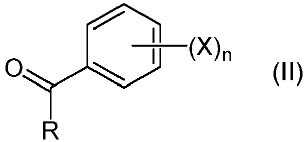

In der Formel (II) weisen R, X und n die bereits genannten Bedeutungen auf.

Die reduktive Alkylierung kann direkt mit molekularem Wasserstoff oder indirekt durch Wasserstoff-Transfer von anderen Reagentien, wie beispielsweise Ameisensäure, erfolgen. Bevorzugt wird molekularer Wasserstoff verwendet. Die Reaktionsbedingungen werden vorteilhaft so gewählt, dass eine oder beide Aminogruppen von 1,2-Propylendiamin mit guter Selektivität einfach alkyliert werden und der Benzolring nicht hydriert wird.
Bevorzugt wird die Umsetzung bei einer Temperatur von 40 bis 120 °C und in Anwesenheit eines geeigneten Katalysators durchgeführt. Als Katalysator bevorzugt sind Palladium auf Kohle (Pd/C), Platin auf Kohle (Pt/C), Adams-Katalysator oder Raney-Nickel, insbesondere Palladium auf Kohle oder Raney-Nickel.
Bei Verwendung von molekularem Wasserstoff wird bevorzugt in einer Druckapparatur bei einem Wasserstoff-Druck von 5 bis 150 bar, insbesondere 10 bis 100 bar, gearbeitet.

Die Herstellung des Amins der Formel (I) durch reduktive Alkylierung auf die beschriebene Weise ist für die Verwendung als Härter für Epoxidharze besonders vorteilhaft, da primäre Aminogruppen mit guter Selektivität einfach alkyliert werden, während sekundäre Aminogruppen kaum weiter alkyliert werden. Das Produkt aus dem beschriebenen Herstellverfahren kann deshalb ohne weitere Aufbereitung als Härter für Epoxidharze in der beschriebenen Weise verwendet werden.

In einer bevorzugten Ausführungsform wird 1,2-Propylendiamin gegenüber dem Aldehyd oder Keton der Formel (II) in einem Molverhältnis von ca. 1/1 eingesetzt. Dabei wird das 1,2-Propylendiamin bevorzugt in einem Lösemittel gelöst, welches nach der Umsetzung destillativ entfernt wird. Diese Herstellung ist besonders ökonomisch. Ein auf diese Weise hergestelltes Amin der Formel (I) weist neben monoalkyliertem 1,2-Propylendiamin (Amin der Formel (I) mit Y für einen Wasserstoff-Rest) einen gewissen Anteil an N,N'-dialkyliertem 1,2-Propylendiamin (Amin der Formel (I) mit Y für einen Rest der Formel 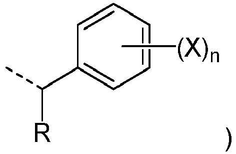 und gegebenenfalls weiteren Alkylierungsprodukten auf. Beispiele für gegebenenfalls enthaltene weitere Alkylierungsprodukte sind in den folgenden Formeln abgebildet.

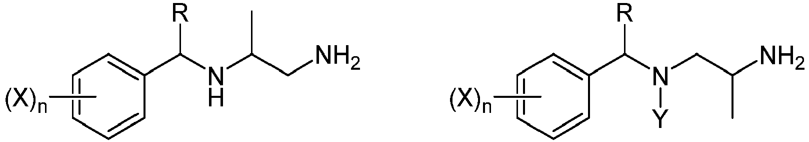

In einer weiteren bevorzugten Ausführungsform wird 1,2-Propylendiamin gegenüber dem Aldehyd oder Keton der Formel (II) im stöchiometrischen Überschuss eingesetzt. Das Molverhältnis zwischen 1,2-Propylendiamin und dem Aldehyd oder Keton der Formel (II) beträgt bevorzugt mindestens 2/1, insbesondere mindestens 3/1. Das überschüssige 1,2-Propylendiamin wird vor oder bevorzugt nach der Reduktion entfernt, insbesondere durch Destillation. Ein auf diese Weise hergestelltes Amin der Formel (I) weist einen besonders hohen Anteil an monoalkyliertem 1,2-Propylendiamin, also Amin der Formel (I) mit Y für einen Wasserstoff-Rest, auf. In der erfindungsgemässen Verwendung zeichnet es sich durch eine besonders starke Verdünnungswirkung aus.

In einer weiteren bevorzugten Ausführungsform wird 1,2-Propylendiamin gegenüber dem Aldehyd oder Keton der Formel (II) im stöchiometrischen Unterschuss eingesetzt. Das Molverhältnis zwischen 1,2-Propylendiamin und dem Aldehyd oder Keton der Formel (II) beträgt dabei bevorzugt 1/1.1 bis 1/2, insbesondere 1/1.2 bis 1/1.9, bevorzugt 1/1.3 bis 1/1.8. Dabei wird 1,2-Propylendiamin bevorzugt in einem Lösemittel gelöst, welches nach der Umsetzung destillativ entfernt wird. Ein auf diese Weise hergestelltes Amin der Formel (I) weist einen erhöhten Anteil an N,N'-dialkyliertem 1,2-Propylendiamin auf. Dies weist den Vorteil auf, dass sein Aminwasserstoff-Equivalentgewicht vergleichsweise hoch ist, wodurch bei gleicher Aminwasserstoff-Dosierung eine besonders gute Verdünnung erzielt wird.

Als Aldehyd der Formel (II) geeignet sind insbesondere Benzaldehyd, 2-Methylbenzaldehyd (o-Tolualdehyd), 3-Methylbenzaldehyd (m-Tolualdehyd), 4-Methylbenzaldehyd (p-Tolualdehyd), 2,5-Dimethylbenzaldehyd, 4-Ethylbenzaldehyd, 4-Isopropylbenzaldehyd (Cuminaldehyd), 4-tert.Butylbenzaldehyd, 2-Methoxybenzaldehyd (o-Anisaldehyd), 3-Methoxybenzaldehyd (m-Anisaldehyd), 4-Methoxybenzaldehyd (Anisaldehyd), 2,3-Dimethoxybenzaldehyd, 2,4-Dimethoxybenzaldehyd, 2,5-Dimethoxybenzaldehyd, 3,4-Dimethoxybenzaldehyd (Veratrumaldehyd), 3,5-Dimethoxybenzaldehyd, 2,4,6-Trimethylbenzaldehyd, 2,4,5-Trimethoxybenzaldehyd (Asaronaldehyd), 2,4,6-Trimethoxybenzaldehyd, 3,4,5-Trimethoxybenzaldehyd oder 4-Dimethylaminobenzaldehyd. Bevorzugt sind Benzaldehyd, 4-Isopropylbenzaldehyd (Cuminaldehyd), 4-tert.Butylbenzaldehyd, 4-Methoxybenzaldehyd (Anisaldehyd) oder 4-Dimethylaminobenzaldehyd.
Als Keton der Formel (II) geeignet sind insbesondere Acetophenon, Benzophenon, 2'-Methylacetophenon, 3'-Methylacetophenon, 4'-Methylacetophenon, 2'-Methoxyacetophenon, 3'-Methoxyacetophenon, 4'-Methoxyacetophenon, 2',4'-Dimethylacetophenon, 2',5'-Dimethylacetophenon, 3',4'-Dimethylacetophenon, 3',5'-Dimethylacetophenon, 2',4'-Dimethoxyacetophenon, 2',5'-Dimethoxyacetophenon, 3',4'-Dimethoxyacetophenon, 3',5'-Dimethoxyacetophenon, 2',4',6'-Trimethylacetophenon oder 2',4',6'-Trimethoxyacetophenon. Bevorzugt sind Acetophenon, Benzophenon, 4'-Methylacetophenon oder 4'-Methoxyacetophenon. Besonders bevorzugt ist Acetophenon.

Als Aldehyd oder Keton der Formel (II) besonders bevorzugt ist Benzaldehyd, 4-Methoxybenzaldehyd oder 4-Dimethylaminobenzaldehyd.
Am meisten bevorzugt ist Benzaldehyd.

In einer Ausführungsform wird eine Mischung aus zwei oder mehr verschiedenen Aldehyden oder Ketonen der Formel (II) für die Umsetzung verwendet, insbesondere eine Mischung aus Benzaldehyd und 4-Methoxybenzaldehyd oder 4-Dimethylaminobenzaldehyd.

Das Amin der Formel (I) wird besonders bevorzugt als Reaktionsprodukt aus der reduktiven Alkylierung von 1,2-Propylendiamin mit mindestens einem Aldehyd oder Keton der Formel (II) und Wasserstoff, wie vorgängig beschrieben, eingesetzt, wobei das 1,2-Propylendiamin gegenüber den Carbonylgruppen des Aldehyds oder Ketons der Formel (II) im stöchiometrischen Überschuss eingesetzt und der Überschuss nach der Reduktion destillativ entfernt wird. Das Verhältnis zwischen der Anzahl 1,2-Propylendiamin-Moleküle und der Anzahl Carbonylgruppen beträgt bevorzugt mindestens 2/1, insbesondere mindestens 3/1, besonders bevorzugt mindestens 4/1.
Ganz besonders bevorzugt wird das Reaktionsprodukt mittels Destillation gereinigt. Dabei wird das Reaktionsprodukt destilliert und das erhaltene Destillat verwendet.
Ein solches Destillat ermöglicht geruchs- und emissionsarme Epoxidharz-Zusammensetzungen mit sehr niedriger Viskosität, schneller Härteentwicklung bzw. Aushärtung und überraschend hoher Härte, welche auch bei feucht-kalten Bedingungen kaum Blushing-bedingte Oberflächenstörungen zeigen und überraschenderweise praktisch nicht vergilben.
Ein solches Destillat besteht typischerweise zur Hauptsache aus dem erfindungsgemässen, am N¹-Stickstoff alkylierten, Produkt und enthält Anteile von am N²-Stickstoff alkylierten Produkt, also insbesondere zur Hauptsache aus N¹-Benzyl-1,2-propandiamin und Anteilen von N²-Benzyl-1,2-propandiamin, oder zur Haupsache aus N¹-(4-Methoxybenzyl)-1,2-propandiamin und Anteilen von N²-(4-Methoxybenzyl)-1,2-propandiamin, oder zur Haupsache aus N¹-(4-(Dimethylamino)benzyl)-1,2-propandiamin und Anteilen von N²-(4-(Dimethylamino)benzyl)-1,2-propandiamin.

In einem Härter für Epoxidharze wird das Amin der Formel (I) bevorzugt in Kombination mit weiteren Aminen und/oder Beschleunigern eingesetzt.

Ein weiterer Gegenstand der Erfindung ist dementsprechend ein Härter für Epoxidharze enthaltend mindestens ein Amin der Formel (I) und mindestens ein weiteres Amin und/oder mindestens einen Beschleuniger. Das weitere Amin ist dabei kein Amin der Formel (I). Ein solcher Härter weist eine besonders hohe Reaktivität gegenüber Epoxidharzen auf.

Als Beschleuniger geeignet sind Substanzen, welche die Reaktion zwischen Aminogruppen und Epoxidgruppen beschleunigen, insbesondere Säuren oder zu Säuren hydrolysierbare Verbindungen, insbesondere organische Carbonsäuren wie Essigsäure, Benzoesäure, Salicylsäure, 2-Nitrobenzoesäure, Milchsäure, organische Sulfonsäuren wie Methansulfonsäure, p-Toluolsulfonsäure oder 4-Dodecylbenzolsulfonsäure, Sulfonsäureester, andere organische oder anorganische Säuren wie insbesondere Phosphorsäure, oder Mischungen der vorgenannten Säuren und Säureester; tertiäre Amine wie insbesondere 1,4-Diazabicyclo[2.2.2]octan, Benzyldimethylamin, α-Methylbenzyldimethylamin, Triethanolamin, Dimethyl-aminopropylamin, Imidazole wie insbesondere N-Methylimidazol, N-Vinylimidazol oder 1,2-Dimethylimidazol, Salze solcher tertiärer Amine, quaternäre Ammoniumsalze, wie insbesondere Benzyltrimethylammoniumchlorid, Amidine wie insbesondere 1,8-Diazabicyclo[5.4.0]undec-7-en, Guanidine wie insbesondere 1,1,3,3-Tetramethylguanidin, Phenole, insbesondere Bisphenole, Phenol-Harze oder Mannich-Basen wie insbesondere 2-(Dimethylaminomethyl)phenol, 2,4,6-Tris(dimethylaminomethyl)phenol oder Polymere aus Phenol, Formaldehyd und N,N-Dimethyl-1,3-propandiamin, Phosphite wie insbesondere Di- oder Triphenylphosphite, oder Mercaptogruppen aufweisende Verbindungen. Als Beschleuniger bevorzugt sind Säuren, tertiäre Amine oder Mannich-Basen.
Am meisten bevorzugt ist Salicylsäure oder 2,4,6-Tris(dimethylaminomethyl)-phenol oder eine Kombination davon.

Als weiteres Amin geeignet sind insbesondere Polyamine, welche mindestens zwei, insbesondere mindestens drei, gegenüber Epoxidgruppen reaktive Aminwasserstoffe aufweisen, insbesondere die folgenden Polyamine:
- Reaktionsprodukte aus der reduktiven Alkylierung von 1,2-Propylendiamin mit einem Aldehyd oder Keton der Formel (II), bei welchen das N²-Stickstoffatom alkyliert ist, wie insbesondere N²-Benzyl-1,2-propandiamin, N²-(4-Methoxybenzyl)-1,2-propandiamin, N²-(4-(Dimethylamino)benzyl)-1,2-propandiamin, N²-(1-Phenylethyl)-1,2-propandiamin, N²-Benzhydryl-1,2-propandiamin, N²-(1-(4'-Methyl)phenylethyl)-1,2-propandiamin oder N²-(1-(4'-Methoxy)phenylethyl)-1,2-propandiamin;
- weitere aliphatische, cycloaliphatische oder arylaliphatische primäre Diamine, insbesondere 2,2-Dimethyl-1,3-propandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2(4),4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3- oder 1,4-Diaminocyclohexan, Bis-(4-aminocyclohexyl)methan (H₁₂-MDA), Bis(4-amino-3-methylcyclohexyl)-methan, Bis(4-amino-3-ethylcyclohexyl)methan, Bis(4-amino-3,5-dimethylcyclohexyl)methan, Bis(4-amino-3-ethyl-5-methylcyclohexyl)methan, 1-Amino-3-aminomethyl-3,5,5-trimethylcyclohexan (Isophorondiamin oder IPDA), 2- oder 4-Methyl-1,3-diaminocyclohexan oder Mischungen davon, 1,3-Bis(aminomethyl)cyclohexan, 1,4-Bis(aminomethyl)cyclohexan, 2,5(2,6)-Bis(aminomethyl)bicyclo[2.2.1]heptan (NBDA), 3(4),8(9)-Bis(aminomethyl)tricyclo-[5.2.1.0^{2,6}]decan, 1,4-Diamino-2,2,6-trimethylcyclohexan (TMCDA), 1,8-Menthandiamin, 3,9-Bis(3-aminopropyl)-2,4,8,10-tetraoxaspiro[5.5]undecan, 1,3-Bis(aminomethyl)benzol (MXDA) oder 1,4-Bis(aminomethyl)benzol;
- aliphatische, cycloaliphatische oder arylaliphatische primäre Triamine, insbesondere 4-Aminomethyl-1,8-octandiamin, 1,3,5-Tris(aminomethyl)benzol, 1,3,5-Tris(aminomethyl)cyclohexan, Tris(2-aminoethyl)amin, Tris(2-aminopropyl)amin oder Tris(3-aminopropyl)amin;
- Ethergruppen-haltige aliphatische primäre Di- oder Triamine, insbesondere Bis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin oder höhere Oligomere dieser Diamine, Bis(3-aminopropyl)polytetrahydrofurane oder andere Polytetrahydrofurandiamine, cycloaliphatische Ethergruppen-haltige Diamine aus der Propoxylierung und nachfolgenden Aminierung von 1,4-Dimethylolcyclohexan, erhältlich insbesondere als Jeffamine® RFD-270 (von Huntsman), oder Polyoxyalkylendi- oder -triamine, welche typischerweise Produkte aus der Aminierung von Polyoxyalkylendi- oder -triolen darstellen und beispielsweise erhältlich sind unter dem Namen Jeffamine® (von Huntsman), unter dem Namen Polyetheramine (von BASF) oder unter dem Namen PC Amine® (von Nitroil). Insbesondere geeignete Polyoxyalkylendi- oder -triamine sind Jeffamine® D-230, Jeffamine® D-400, Jeffamine® D-2000, Jeffamine® EDR-104, Jeffamine® EDR-148, Jeffamine® EDR-176, Jeffamine® T-403, Jeffamine® T-3000, Jeffamine® T-5000, oder entsprechende Amine von BASF oder Nitroil;
- sekundäre Aminogruppen aufweisende Polyamine mit zwei primären aliphatischen Aminogruppen, wie insbesondere 3-(2-Aminoethyl)aminopropylamin, Bis(hexamethylen)triamin (BHMT), Diethylentriamin (DETA), Triethylentetramin (TETA), Tetraethylenpentamin (TEPA), Pentaethylenhexamin (PEHA) oder höhere Homologe linearer Polyethylenamine wie Polyethylenpolyamin mit 5 bis 7 Ethylenamin-Einheiten (sogenanntes "higher ethylenepolyamine", HEPA), Produkte aus der mehrfachen Cyanoethylierung oder Cyanobutylierung und anschliessender Hydrierung von primären Di- und Polyaminen mit mindestens zwei primären Aminogruppen, wie Dipropylentriamin (DPTA), N-(2-Aminoethyl)-1,3-propandiamin (N3-Amin), N,N'-Bis(3-aminopropyl)ethylendiamin (N4-Amin), N,N'-Bis(3-aminopropyl)-1,4-diaminobutan, N5-(3-Aminopropyl)-2-methyl-1,5-pentandiamin, N3-(3-Aminopentyl)-1,3-pentandiamin, N5-(3-Amino-1-ethylpropyl)-2-methyl-1,5-pentandiamin oder N,N'-Bis(3-amino-1-ethylpropyl)-2-methyl-1,5-pentandiamin;
- Polyamine mit ein oder zwei sekundären Aminogruppen, insbesondere Produkte aus der reduktiven Alkylierung von primären aliphatischen Polyaminen mit Aldehyden oder Ketonen, insbesondere N-Benzyl-1,3-bis(aminomethyl)-benzol, N,N'-Dibenzyl-1,3-bis(aminomethyl)benzol, N-2-Ethylhexyl-1,3-bis-(aminomethyl)benzol, N,N'-Bis(2-ethylhexyl)-1,3-bis(aminomethyl)benzol, oder partiell styrolisierte Polyamine wie zum Beispiel styrolisiertes MXDA (erhältlich als Gaskamine® 240 von Mitsubishi Gas Chemical);
- aromatische Polyamine, wie insbesondere m- und p-Phenylendiamin, 4,4'-, 2,4' und/oder 2,2'-Diaminodiphenylmethan, 3,3'-Dichloro-4,4'-diaminodiphenylmethan (MOCA), 2,4- und/oder 2,6-Toluylendiamin, Mischungen von 3,5-Dimethylthio-2,4- und -2,6-toluylendiamin (erhältlich als Ethacure® 300 von Albermarle), Mischungen von 3,5-Diethyl-2,4- und -2,6-toluylendiamin (DETDA), 3,3',5,5'-Tetraethyl-4,4'-diaminodiphenylmethan (M-DEA), 3,3',5,5'-Tetraethyl-2,2'-dichloro-4,4'-diaminodiphenylmethan (M-CDEA), 3,3'-Diisopropyl-5,5'-dimethyl-4,4'-diaminodiphenylmethan (M-MIPA), 3,3',5,5'-Tetraisopropyl-4,4'-diaminodiphenylmethan (M-DIPA), 4,4'-Diaminodiphenylsulfon (DDS), 4-Amino-N-(4-aminophenyl)benzolsulfonamid, 5,5'-Methylendianthranilsäure, Dimethyl-(5,5'-methylendianthranilat), 1,3-Propylen-bis(4-aminobenzoat), 1,4-Butylen-bis(4-aminobenzoat), Polytetramethylenoxid-bis(4-aminobenzoat) (erhältlich als Versalink® von Air Products), 1,2-Bis(2-aminophenylthio)ethan, 2-Methylpropyl-(4-chloro-3,5-diaminobenzoat) oder tert.Butyl-(4-chloro-3,5-diaminobenzoat);
- Addukte der genannten Polyamine mit Epoxiden oder Epoxidharzen, insbesondere Addukte mit Diepoxiden im Molverhältnis von ungefähr 2/1, Addukte mit Monoepoxiden im Molverhältnis von ungefähr 1/1, oder Umsetzungsprodukte aus Aminen und Epichlorhydrin, insbesondere jenes von 1,3-Bis(aminomethyl)benzol, kommerziell erhältlich als Gaskamine® 328 (von Mitsubishi Gas Chemical);
- Polyamidoamine, insbesondere Umsetzungsprodukte aus einer ein- oder mehrwertigen Carbonsäure, beziehungsweise deren Ester oder Anhydride, insbesondere einer Dimerfettsäure, mit einem im stöchiometrischen Überschuss eingesetzten aliphatischen, cycloaliphatischen oder aromatischen Polyamin, insbesondere einem Polyalkylenamin wie beispielsweise DETA oder TETA, insbesondere die kommerziell erhältlichen Polyamidoamine Versamid® 100, 125, 140 oder 150 (von Cognis), Aradur® 223, 250 oder 848 (von Huntsman), Euretek® 3607 oder 530 (von Huntsman) oder Beckopox® EH 651, EH 654, EH 655, EH 661 oder EH 663 (von Cytec); oder
- Phenalkamine, auch Mannich-Basen genannt, insbesondere Umsetzungsprodukte aus einer Mannich-Reaktion von Phenolen, insbesondere Cardanol, mit Aldehyden, insbesondere Formaldehyd, insbesondere die kommerziell erhältlichen Phenalkamine Cardolite® NC-541, NC-557, NC-558, NC-566, Lite 2001, Lite 2002, NX-4943, NX-5607 oder NX-5608 (von Cardolite), Aradur® 3440, 3441, 3442 oder 3460 (von Huntsman) oder Beckopox® EH 614, EH 621, EH 624, EH 628 oder EH 629 (von Cytec).

Als weiteres Amin bevorzugt sind Reaktionsprodukte aus der reduktiven Alkylierung von 1,2-Propylendiamin mit einem Aldehyd oder Keton der Formel (II), bei welchen das N²-Stickstoffatom alkyliert ist, insbesondere N²-Benzyl-1,2-propandiamin. Ein solches Amin ist insbesondere als Bestandteil eines Reaktionsprodukts enthaltend das entsprechende N¹-alkylierte Amin vorhanden.

Als weiteres Amin bevorzugt sind weiterhin Addukte aus (i) mindestens einem Polyamin mit mindestens drei gegenüber Epoxidgruppen reaktiven Aminwasserstoffen mit (ii) mindestens einem Epoxid.
Als Polyamin für ein solches Addukt bevorzugt sind die vorgängig genannten Polyamine mit mindestens drei gegenüber Epoxidgruppen reaktiven Aminwasserstoffen, oder kleinere Polyamine wie insbesondere Ethylendiamin, die isomeren Propylendiamine oder die isomeren Butylendiamine.
Als Epoxid für ein solches Addukt bevorzugt sind Diepoxide, wie insbesondere Bisphenol-A- oder -F- oder -A/F-diglycidylether, Poly-1,2-propylenoxiddiglycidylether oder Monoepoxide. Besonders bevorzugt sind aromatische Monoepoxide, insbesondere Kresylglycidylether, tert.Butylphenylglycidylether oder der Glycidylether von Cardanol. Besonders bevorzugt ist Kresylglycidylether. Als Kresylglycidylether geeignet sind alle isomeren Kresylglycidylether oder Gemische davon, insbesondere kommerziell erhältiche Typen wie insbesondere Araldite® DY-K (von Huntsman), Polypox™ R6 (von Dow), Heloxy™ KR (von Hexion) oder Erisys® GE-10 (von CVC Spec. Chem.).
Das Addukt wird bevorzugt hergestellt durch langsames Zudosieren des Epoxids zu vorgelegtem Polyamin, wobei die Temperatur der Reaktanden bevorzugt im Bereich von 40 bis 120 °C, insbesondere 50 bis 110 °C, gehalten wird. Solche Addukte zeigen ausgezeichnete Eigenschaften als Härter für Epoxidharze, insbesondere eine schnelle Aushärtungsgeschwindigkeit auch bei tiefen Temperaturen und eine wenig ausgeprägte Neigung zu Blushing-Effekten. Sie ergeben Filme von ausgezeichneter Qualität, sind aber aufgrund ihrer Viskosität für Beschichtungsanwendungen nur geeignet, wenn sie verdünnt werden. Durch die Kombination mit einem Amin der Formel (I) wird das Addukt soweit verdünnt, dass Härter für emissionsarme Epoxidharz-Beschichtungen mit hervorragenden Eigenschaften zugänglich sind.

Bevorzugt sind Addukte aus (i) mindestens einem Polyamin mit mindestens drei gegenüber Epoxidgruppen reaktive Aminwasserstoffen mit (ii) mindestens einem aromatischen Monoepoxid, welche im Molverhältnis von ungefähr 1/1 umgesetzt sind. Während der Umsetzung kann das Polyamin im Überschuss vorhanden gewesen und nach der Umsetzung mittels Destillation entfernt worden sein.
Für ein solches Addukt ist das Polyamin bevorzugt ausgewählt aus der Gruppe bestehend aus Ethylendiamin, 1,2-Propylendiamin, 1,3-Propylendiamin, 1,4-Butylendiamin, 1,3-Butylendiamin, 1,2-Butylendiamin, 2,3- Butylendiamin, 2-Methyl-1,3-propandiamin, DAMP, 2,2-Dimethyl-1,3-propandiamin, 1,5-Pentandiamin, MPMD, 1,6-Hexandiamin, 2,5-Dimethyl-1,6-hexandiamin, TMD, 1,2-Diaminocyclohexan, 1,3-Diaminocyclohexan, 1,4-Diaminocyclohexan, IPDA, 2-Methyl-1,3-diaminocyclohexan und 4-Methyl-1,3-diaminocyclohexan und Mischungen davon, 1,3-Bis(aminomethyl)cyclohexan, 1,4-Bis(aminomethyl)cyclohexan, 1,3-Bis(aminomethyl)benzol, Bis(2-aminoethyl)ether, 3,6-Dioxaoctan-1,8-diamin, DETA, TETA, DPTA, N3-Amin, N4-Amin und BHMT.
Für ein solches Addukt ist das aromatische Monoepoxid bevorzugt ein Kresylglycidylether.

Besonders bevorzugt ist ein Addukt aus 1,2-Propylendiamin mit Kresylglycidylether, welches hergestellt ist mit einem Überschuss 1,2-Propylendiamin und nachfolgender Entfernung des Überschusses mittels Destillation.
Weiterhin besonders bevorzugt ist ein Addukt aus 1,5-Diamino-2-methylpentan mit Kresylglycidylether, welches entweder hergestellt ist mit einem Überschuss 1,5-Diamino-2-methylpentan und nachfolgender Entfernung des Überschusses mittels Destillation, oder mit einem leichten Überschuss an Kresylglycidylether. Weiterhin besonders bevorzugt ist ein Addukt aus 2,2(4),4-Trimethylhexamethylendiamin mit Kresylglycidylether, welches hergestellt ist mit einem leichten Überschuss an 2,2(4),4-Trimethylhexamethylendiamin.

Der Begriff "Überschuss" bezieht sich bei diesen besonders bevorzugten Addukten nicht auf die Reaktivgruppen, sondern auf das Molverhältnis zwischen dem Polyaminmolekül und dem Kresylglyciylether.
Diese besonders bevorzugten Addukte sind vergleichsweise niedrigviskos, weisen eine besonders gute Verträglichkeit und Reaktivität mit den üblichen Epoxidharz-Zusammensetzungen auf, neigen kaum zu Blushing-Effekten und ermöglichen ausgehärtete Filme von hohem Glanz und hoher Härte. Ohne einen verdünnenden Zusatz sind aber auch diese Addukte zu hochviskos als Härter für Epoxidharz-Beschichtungen.

Als weiteres Amin bevorzugt sind weiterhin Ethergruppen-haltige aliphatische primäre Di- oder Triamine, insbesondere Polyoxyalkylendi- oder -triamine mit einem mittleren Molekulargewicht im Bereich von 200 bis 500 g/mol, insbesondere Jeffamine® D-230 oder Jeffamine® T-403 (beide von Huntsman), oder cycloaliphatische Ethergruppen-haltige Diamine aus der Propoxylierung und nachfolgenden Aminierung von 1,4-Dimethylolcyclohexan, insbesondere Jeffamine® RFD-270 (von Huntsman). Ein solches Amin ermöglicht Epoxidharz-Zusammensetzungen mit einer zuverlässigen Aushärtung zu hoher Endhärte ohne sogenanntes "Einfrieren" und mit geringer Sprödigkeit nach der Aushärtung. Mit "Einfrieren" wird das Phänomen bezeichnet, dass eine Epoxidharz-Zusammensetzung bei einer gegebenen Temperatur nach anfänglich guter Härteentwicklung nicht zu der erwarteten Endhärte aushärtet, sondern die Aushärtung bei einer geringeren Härte stehenbleibt. Solche Effekte treten insbesondere bei tiefen Aushärtungstemperaturen auf.

Der erfindungsgemässe Härter enthält bevorzugt 1 bis 95 Gewichts-%, bevorzugt 2 bis 80 Gewichts-%, besonders bevorzugt 5 bis 60 Gewichts-%, insbesondere 5 bis 40 Gewichts-%, Amin der Formel (I). Solche Härter zeichnen sich durch eine niedrige Viskosität aus und ermöglichen Epoxidharz-Beschichtungen mit hoher Aushärtungsgeschwindigkeit, kaum Neigung zu Blushing-Effekten und hoher Härte.

Typischerweise enthält der Härter einen gewissen Anteil an am N²-Stickstoff alkylierten Produkten, beispielsweise im Fall von N¹-Benzyl-1,2-propandiamin einen gewissen Anteil an N²-Benzyl-1,2-propandiamin.

Ein besonders bevorzugter Härter für Epoxidharze enthält
- mindestens ein Amin der Formel (I),
- mindestens ein Addukt aus (i) mindestens einem Polyamin mit mindestens drei gegenüber Epoxidgruppen reaktive Aminwasserstoffen und (ii) mindestens einem aromatischen Monoepoxid, und
- mindestens ein Ethergruppen-haltiges aliphatisches primäres Di- oder Triamin.
Dabei liegen das Amin der Formel (I), das Addukt und das Ethergruppen-haltige Di- oder Triamin insbesondere in einer solchen Menge vor, dass von den gesamten Aminwasserstoffen des Härters
10 bis 40 % aus dem Amin der Formel (I),
15 bis 75 % aus dem Addukt, und
15 bis 60 % aus dem Ethergruppen-haltigen Di- oder Triamin stammen.
Ein solcher Härter weist eine niedrige Viskosität auf und härtet schnell und weitgehend ohne Blushing-Effekte zu ausgehärteten Filmen von hohem Glanz und hoher Härte, welche kaum vergilben.

Ein weiterer besonders bevorzugter Härter für Epoxidharze enthält
- mindestens ein Amin der Formel (I),
- mindestens ein Addukt aus (i) mindestens einem Polyamin mit mindestens drei gegenüber Epoxidgruppen reaktive Aminwasserstoffen und (ii) mindestens einem aromatischen Monoepoxid, und
- gegebenenfalls mindestens ein weiteres Amin.
Dabei liegen das Amin der Formel (I), das Addukt und das weitere Amin in einer solchen Menge vor, dass von den gesamten Aminwasserstoffen des Härters
10 bis 80 % aus dem Amin der Formel (I),
20 bis 80 % aus dem Addukt, und
0 bis 40 % aus mindestens einem weiteren Amin stammen.

Ein solcher Härter weist eine niedrige Viskosität auf und härtet besondes schnell und weitgehend ohne Blushing-Effekte zu ausgehärteten Filmen von hohem Glanz und hoher Härte, welche kaum vergilben.

Der Härter ist bevorzugt weitgehend frei von 1,2-Propylendiamin. Er enthält insbesondere weniger als 1 Gewichts-%, besonders bevorzugt weniger als 0.1 Gewichts-%, 1,2-Propylendiamin.
Weiterhin bevorzugt ist der Härter weitgehend frei von Aminen mit einem Molekulargewicht unterhalb von 120 g/mol, insbesondere unterhalb von 150 g/mol. Bevorzugt enthält der Härter weniger als 2 Gewichts-%, insbesondere weniger als 1 Gewichts-%, Amine mit einem Molekulargewicht unterhalb von 120 g/mol, insbesondere unterhalb von 150 g/mol.
Ein solcher Härter ist toxikologisch und geruchlich besonders vorteilhaft und ermöglicht Beschichtungen mit besonders schönen Oberflächen.

Der Härter kann weiterhin mindestens einen nicht einbaubaren Verdünner enthalten, insbesondere Xylol, 2-Methoxyethanol, Dimethoxyethanol, 2-Ethoxyethanol, 2-Propoxyethanol, 2-Isopropoxyethanol, 2-Butoxyethanol, 2-Phenoxyethanol, 2-Benzyloxyethanol, Benzylalkohol, Ethylenglykol, Ethylenglykoldimethylether, Ethylenglykoldiethylether, Ethylenglykoldibutylether, Ethylenglykoldiphenylether, Diethylenglykol, Diethylenglykol-monomethylether, Diethylenglykol-monoethylether, Diethylenglykol-mono-n-butylether, Diethylenglykoldimethylether, Diethylenglykoldiethylether, Diethylenglykoldi-n-butylylether, Propylenglykolbutylether, Propylenglykolphenylether, Dipropylenglykol, Dipropylenglykolmonomethylether, Dipropylenglykoldimethylether, Dipropylenglykoldi-n-butylether, N-Methylpyrrolidon, Diphenylmethan, Diisopropylnaphthalin, Erdölfraktionen wie zum Beispiel Solvesso®-Typen (von Exxon), Alkylphenole wie tert.Butylphenol, Nonylphenol, Dodecylphenol und 8,11,14-Pentadecatrienylphenol (Cardanol, aus Cashewschalen-Öl, erhältlich beispielsweise als Cardolite NC-700 von Cardolite Corp., USA), styrolisiertes Phenol, Bisphenole, aromatische Kohlenwasserstoffharze, insbesondere Phenolgruppen-haltige Typen, alkoxyliertes Phenol, insbesondere ethoxyliertes oder propoxyliertes Phenol, insbesondere 2-Phenoxyethanol, Adipate, Sebacate, Phthalate, Benzoate, organische Phosphor- oder Sulfonsäureester oder Sulfonamide. Bevorzugt sind Benzylalkohol, Dodecylphenol, tert.Butylphenol, styrolisiertes Phenol, ethoxyliertes Phenol oder phenolgruppenhaltige aromatische Kohlenwasserstoffharze, insbesondere die Novares®-Typen LS 500, LX 200, LA 300 oder LA 700 (von Rütgers).

Bevorzugt enthält der Härter keinen oder nur einen geringen Gehalt an nicht einbaubaren Verdünnern. Bevorzugt enthält der Härter maximal 5 Gewichts-% nicht einbaubare Verdünner.

Der Härter kann weitere gegenüber Epoxidgruppen reaktive Substanzen enthalten, beispielsweise Monoamine wie Hexylamin oder Benzylamin, oder Mercaptogruppen aufweisende Verbindungen, insbesondere die Folgenden:
- flüssige Mercaptan-terminierte Polysulfid-Polymere, bekannt unter dem Markennamen Thiokol® (von Morton Thiokol; beispielsweise erhältlich von SPI Supplies, oder von Toray Fine Chemicals), insbesondere die Typen LP-3, LP-33, LP-980, LP-23, LP-55, LP-56, LP-12, LP-31, LP-32 oder LP-2; sowie weiterhin bekannt unter dem Markennamen Thioplast® (von Akzo Nobel), insbesondere die Typen G 10, G 112, G 131, G 1, G 12, G 21, G 22, G 44 oder G 4;
- Mercaptan-terminierte Polyoxyalkylen-Ether, erhältlich beispielsweise durch Umsetzung von Polyoxyalkylendi- oder -triolen entweder mit Epichlorhydrin oder mit einem Alkylenoxid, gefolgt von Natriumhydrogensulfid;
- Mercaptan-terminierte Verbindungen in Form von Polyoxyalkylen-Derivaten, bekannt unter dem Markennamen Capcure® (von Cognis), insbesondere die Typen WR-8, LOF oder 3-800;
- Polyester von Thiocarbonsäuren, beispielsweise Pentaerythritoltetramercaptoacetat, Trimethylolpropantrimercaptoacetat, Glykoldimercaptoacetat, Pentaerythritoltetra-(3-mercaptopropionat), Trimethylolpropantri(3-mercaptopropionat) oder Glykoldi-(3-mercaptopropionat), oder Veresterungsprodukte von Polyoxyalkylendiolen oder -triolen, ethoxyliertem Trimethylolpropan oder Polyester-Diolen mit Thiocarbonsäuren wie Thioglykolsäure oder 2- oder 3-Mercaptopropionsäure; oder
- weitere Mercaptogruppen aufweisende Verbindungen, wie insbesondere 2,4,6-Trimercapto-1,3,5-triazin, 2,2'-(Ethylendioxy)-diethanthiol (Triethylenglykol-dimercaptan) oder Ethandithiol.

Ein weiterer Gegenstand der Erfindung ist eine Epoxidharz-Zusammensetzung umfassend
- eine Harz-Komponente enthaltend mindestens ein Epoxidharz und
- eine Härter-Komponente enthaltend mindestens ein Amin der Formel (I) wie vorgängig beschrieben.
Bevorzugt enthält die Härter-Komponente einen Härter enthaltend mindestens ein Amin der Formel (I) und mindestens ein weiteres Amin und/oder mindestens einen Beschleuniger, wie vorgängig beschrieben.

Als Epoxidharz sind übliche technische Epoxidharze geeignet. Diese werden auf bekannte Art und Weise erhalten, zum Beispiel aus der Oxidation der entsprechenden Olefine oder aus der Reaktion von Epichlorhydrin mit den entsprechenden Polyolen, Polyphenolen oder Aminen.
Als Epoxidharz besonders geeignet sind sogenannte Polyepoxid-Flüssigharze, im folgenden als "Flüssigharz" bezeichnet. Diese weisen eine Glasübergangstemperatur unterhalb von 25°C auf.
Ebenfalls möglich als Epoxidharz sind sogenannte Festharze, welche eine Glasübergangstemperatur oberhalb von 25°C aufweisen und sich zu bei 25°C schüttfähigen Pulvern zerkleinern lassen.

Geeignete Epoxidharze sind insbesondere aromatische Epoxidharze, insbesondere die Glycidylisierungsprodukte von:
- Bisphenol-A, Bisphenol-F oder Bisphenol-A/F, wobei A für Aceton und F für Formaldehyd steht, welche als Edukte zur Herstellung dieser Bisphenole dienten. Im Fall von Bisphenol-F können auch Stellungsisomere vorhanden sein, insbesondere abgeleitet von 2,4'- oder 2,2'-Hydroxyphenylmethan.
- Dihydroxybenzol-Derivaten wie Resorcin, Hydrochinon oder Brenzkatechin;
- weiteren Bisphenolen oder Polyphenolen wie Bis(4-hydroxy-3-methylphenyl)methan, 2,2-Bis(4-hydroxy-3-methylphenyl)propan (Bisphenol-C), Bis-(3,5-dimethyl-4-hydroxyphenyl)methan, 2,2-Bis(3,5-dimethyl-4-hydroxyphenyl)propan, 2,2-Bis(3,5-dibromo-4-hydroxyphenyl)propan, 2,2-Bis(4-hydroxy-3-tert.butylphenyl)propan, 2,2-Bis(4-hydroxyphenyl)butan (Bisphenol-B), 3,3-Bis(4-hydroxyphenyl)pentan, 3,4-Bis(4-hydroxyphenyl)hexan, 4,4-Bis(4-hydroxyphenyl)heptan, 2,4-Bis(4-hydroxyphenyl)-2-methylbutan, 2,4-Bis-(3,5-dimethyl-4-hydroxyphenyl)-2-methylbutan, 1,1-Bis(4-hydroxyphenyl)-cyclohexan (Bisphenol-Z), 1,1-Bis(4-hydroxyphenyl)-3,3,5-trimethylcyclohexan (Bisphenol-TMC), 1,1-Bis(4-hydroxyphenyl)-1-phenylethan, 1,4-Bis[2-(4-hydroxyphenyl)-2-propyl]benzol (Bisphenol-P), 1,3-Bis[2-(4-hydroxyphenyl)-2-propyl]benzol (Bisphenol-M), 4,4'-Dihydroxydiphenyl (DOD), 4,4'-Dihydroxybenzophenon, Bis(2-hydroxynaphth-1-yl)methan, Bis(4-hydroxynaphth-1-yl)methan, 1,5-Dihydroxynaphthalin, Tris(4-hydroxyphenyl)methan, 1,1,2,2-Tetrakis(4-hydroxyphenyl)ethan, Bis(4-hydroxyphenyl)ether oder Bis(4-hydroxyphenyl)sulfon;
- Kondensationsprodukten von Phenolen mit Formaldehyd, die unter sauren Bedingungen erhalten werden, wie Phenol-Novolaken oder Kresol-Novolaken, auch Bisphenol-F-Novolake genannt;
- aromatischen Aminen, wie Anilin, Toluidin, 4-Aminophenol, 4,4'-Methylendiphenyldiamin, 4,4'-Methylendiphenyldi-(N-methyl)amin, 4,4'-[1,4-Phenylen-bis(1-methylethyliden)]bisanilin(Bisanilin-P) oder 4,4'-[1,3-Phenylen-bis(1-methylethyliden)]bisanilin (Bisanilin-M).

Weitere geeignete Epoxidharze sind aliphatische oder cycloaliphatische Polyepoxide, insbesondere
- Glycidylether von gesättigten oder ungesättigten, verzweigten oder unverzweigten, zyklischen oder offenkettigen di-, tri- oder tetrafunktionellen C₂-bis C₃₀-Alkoholen, insbesondere Ethylenglykol, Propylenglykol, Butylenglykol, Hexandiol, Octandiol, Polypropylenglykolen, Dimethylolcyclohexan, Neopentylglykol, Dibromoneopentylglykol, Rizinusöl, Trimethylolpropan, Trimethylolethan, Pentaerythrol, Sorbit oder Glycerin, oder alkoxyliertes Glycerin oder alkoxyliertes Trimethylolpropan;
- ein hydriertes Bisphenol-A-, -F- oder -A/F-Flüssigharz, beziehungsweise die Glycidylisierungsprodukte von hydriertem Bisphenol-A, -F oder -A/F;
- ein N-Glycidylderivat von Amiden oder heterocyclischen Stickstoffbasen, wie Triglycidylcyanurat oder Triglycidylisocyanurat, oder Umsetzungsprodukte von Epichlorhydrin mit Hydantoin.
- Epoxidharze aus der Oxidation von Olefinen, wie insbesondere Vinylcylohexen, Dicyclopentadien, Cyclohexadien, Cyclododecadien, Cyclododecatrien, Isopren, 1,5-Hexadien, Butadien, Polybutadien oder Divinylbenzol.

Als Epoxidharz in der Harz-Komponente bevorzugt ist ein Flüssigharz auf der Basis eines Bisphenols, insbesondere ein Diglycidylether von Bisphenol-A, Bisphenol-F oder Bisphenol-A/F, wie sie kommerziell beispielsweise von Dow, Huntsman oder Momentive erhältlich sind. Diese Flüssigharze weisen eine für Epoxidharze niedrige Viskosität und im ausgehärteten Zustand gute Eigenschaften als Beschichtung auf. Sie können Anteile von Bisphenol A-Festharz oder Bisphenol-F-Novolaken enthalten.

Die Harz-Komponente kann einen Reaktivverdünner, insbesondere einen mindestens eine Epoxidgruppe aufweisenden Reaktivverdünner, enthalten. Als Reaktivverdünner geeignet sind insbesondere die Glycidylether von ein- oder mehrwertigen Phenolen oder aliphatischen oder cycloaliphatischen Alkoholen, wie insbesondere die bereits genannten Polyglycidylether von Di- oder Polyolen, oder weiterhin Phenylglycidylether, Kresylglycidylether, Benzylglycidylether, p-n-Butylphenylglycidylether, p-tert.Butylphenylglycidylether, Nonylphenylglycidylether, Allylglycidylether, Butylglycidylether, Hexylglycidylether, 2-Ethylhexylglycidylether, oder Glycidylether von natürlichen Alkoholen wie insbesondere C₈- bis C₁₀-Alkylglycidylether oder C₁₂- bis C₁₄-Alkylglycidylether. Die Zugabe eines Reaktivverdünners zum Epoxidharz bewirkt eine Reduktion der Viskosität, und/oder eine Reduktion der Glasübergangstemperatur und/oder der mechanischen Werte.

Gegebenenfalls enthält die Epoxidharz-Zusammensetzung weitere Bestandteile, insbesondere in Epoxidharz-Zusammensetzungen üblicherweise eingesetzte Hilfs- und Zusatzstoffe, beispielsweise die Folgenden:
- Lösemittel, Verdünner, Filmbildehilfsmittel oder Extender, wie insbesondere die bereits genannten nicht einbaubaren Verdünner;
- Reaktivverdünner, insbesondere Epoxidgruppen aufweisende Reaktivverdünner, wie sie vorgängig erwähnt wurden, epoxidiertes Sojaöl oder Leinöl, Acetoacetatgruppen aufweisende Verbindungen, insbesondere acetoacetylierte Polyole, Butyrolakton, Carbonate, Aldehyde, sowie weiterhin Isocyanate oder Reaktivgruppen-aufweisende Silikone;
- Polymere, insbesondere Polyamide, Polysulfide, Polyvinylformal (PVF), Polyvinylbutyral (PVB), Polyurethane (PUR), Polymere mit Carboxylgruppen, Polyamide, Butadien-Acrylnitril-Copolymere, Styrol-Acrylnitril-Copolymere, Butadien-Styrol-Copolymere, Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere chlorsulfonierte Polyethylene oder Fluor-haltige Polymere, Sulfonamid-modifizierte Melamine oder gereinigte Montan-Wachse;
- anorganische oder organische Füllstoffe, insbesondere gemahlene oder gefällte Calciumcarbonate, welche gegebenenfalls mit Fettsäuren, insbesondere Stearaten, beschichtet sind, Baryt (Schwerspat), Talke, Quarzmehle, Quarzsand, Eisenglimmer, Dolomite, Wollastonite, Kaoline, Mica (Kalium-Aluminium-Silikat), Molekularsiebe, Aluminiumoxide, Aluminiumhydroxide, Magnesiumhydroxid, Kieselsäuren, Zemente, Gipse, Flugaschen, Russ, Graphit, Metall-Pulver wie Aluminium, Kupfer, Eisen, Zink, Silber oder Stahl, PVC-Pulver oder Hohlkugeln;
- Fasern, insbesondere Glasfasern, Kohlefasern, Metallfasern, Keramikfasern oder Kunststofffasern wie Polyamidfasern oder Polyethylenfasern;
- Pigmente, insbesondere Titandioxid und/oder Eisenoxide;
- die vorgenannten Beschleuniger;
- Rheologie-Modifizierer, insbesondere Verdicker oder Antiabsetzmittel;
- Haftverbesserer, insbesondere Organoalkoxysilane;
- Stabilisatoren gegen Oxidation, Wärme, Licht oder UV-Strahlung;
- flammhemmende Substanzen, insbesondere Aluminiumhydroxid (ATH), Magnesiumdihydroxid (MDH), Antimontrioxid, Antimonpentoxid, Borsäure (B(OH)₃), Zinkborat, Zinkphosphat, Melaminborat, Melamincyanurat, Ammoniumpolyphosphat, Melaminphosphat, Melaminpyrophosphat, polybromierte Diphenyloxide oder Diphenylether, Phosphate wie insbesondere Diphenylkresylphosphat, Resorcinol-bis(diphenylphosphat), Resorcinoldiphosphat-Oligomer, Tetraphenylresorcinoldiphosphit, Ethylendiamindiphosphat oder Bisphenol-A-bis(diphenylphosphat), Tris(chloroethyl)phosphat, Tris(chloropropyl)phosphat oder Tris(dichloroisopropyl)phosphat, Tris[3-bromo-2,2-bis-(bromomethyl)propyl]phosphat, Tetrabromo-Bisphenol-A, Bis(2,3-dibromopropylether) von Bisphenol A, bromierte Epoxidharze, Ethylen-bis(tetrabromophthalimid), Ethylen-bis(dibromonorbornandicarboximid), 1,2-Bis(tribromophenoxy)ethan, Tris(2,3-dibromopropyl)isocyanurat, Tribromophenol, Hexabromocyclododecan, Bis(hexachlorocyclopentadieno)cyclooctan oder Chlorparaffine;
- oberflächenaktive Substanzen, insbesondere Netzmittel, Verlaufsmittel, Entlüftungsmittel oder Entschäumer;
- Biozide, wie beispielsweise Algizide, Fungizide oder das Pilzwachstum hemmende Substanzen.

Bevorzugt enthält die Epoxidharz-Zusammensetzung weitere Hilfs- und Zusatzstoffe, insbesondere Netzmittel, Verlaufsmittel, Entschäumer, Stabilisatoren, Pigmente und/oder Beschleuniger, insbesondere Salicylsäure und/oder 2,4,6-Tris(dimethylaminomethyl)phenol.
Bevorzugt enthält die Epoxidharz-Zusammensetzung keinen oder nur einen geringen Gehalt an nicht einbaubaren Verdünnern, bevorzugt maximal 5 Gewichts-%, insbesondere maximal 2 Gewichts-%.

In der Epoxidharz-Zusammensetzung liegt das Verhältnis der Anzahl von gegenüber Epoxidgruppen reaktiven Gruppen gegenüber der Anzahl Epoxidgruppen bevorzugt im Bereich von 0.5 bis 1.5, insbesondere 0.7 bis 1.2.
Die in der Epoxidharz-Zusammensetzung vorhandenen Aminwasserstoffe und gegebenenfalls vorhandene weitere gegenüber Epoxidgruppen reaktive Gruppen reagieren mit den Epoxidgruppen unter deren Ringöffnung (Additionsreaktion). Als Ergebnis dieser Reaktionen polymerisiert die Zusammensetzung und härtet schliesslich aus. Dem Fachmann ist bekannt, dass primäre Aminogruppen gegenüber Epoxidgruppen difunktionell sind und eine primäre Aminogruppe somit als zwei gegenüber Epoxidgruppen reaktive Gruppen zählt.

Die beiden Komponenten der Epoxidharz-Zusammensetzung werden jeweils in einem eigenen Gebinde gelagert. Weitere Bestandteile der Epoxidharz-Zusammensetzung können als Bestandteil der Harz- oder der Härter-Komponente vorhanden sein, wobei gegenüber Epoxidgruppen reaktive weitere Bestandteile bevorzugt ein Bestandteil der Härter-Komponente sind. Ein geeignetes Gebinde zum Lagern der Harz- oder der Härter-Komponente ist insbesondere ein Fass, ein Hobbock, ein Beutel, ein Eimer, eine Büchse, eine Kartusche oder eine Tube. Die Komponenten sind lagerfähig, das heisst, dass sie vor ihrer Anwendung während mehreren Monaten bis zu einem Jahr und länger aufbewahrt werden können, ohne dass sie sich in ihren jeweiligen Eigenschaften in einem für ihren Gebrauch relevanten Ausmass verändern. Zur Anwendung der Epoxidharz-Zusammensetzung werden die Harz- und die Härter-Komponente kurz vor oder während der Applikation miteinander vermischt. Das Mischungsverhältnis zwischen den beiden Komponenten wird bevorzugt so gewählt, dass die gegenüber Epoxidgruppen reaktiven Gruppen der Härter-Komponente in einem geeigneten Verhältnis zu den Epoxidgruppen der Harz-Komponente stehen, wie vorgängig beschrieben. In Gewichtsteilen liegt das Mischungsverhältnis zwischen der Harz-Komponente und der Härter-Komponente üblicherweise im Bereich von 1:10 bis 10:1.
Die Vermischung der beiden Komponenten erfolgt mittels eines geeigneten Verfahrens; sie kann kontinuierlich oder batchweise erfolgen. Falls das Vermischen vor der Applikation erfolgt, muss darauf geachtet werden, dass zwischen dem Vermischen der Komponenten und der Applikation nicht zu viel Zeit vergeht, da es dadurch zu Störungen, wie beispielsweise einem verlangsamten oder unvollständigen Aufbau der Haftung zum Substrat, kommen kann. Die Vermischung erfolgt insbesondere bei Umgebungstemperatur, welche typischerweise im Bereich von etwa 5 bis 50°C, bevorzugt bei etwa 10 bis 30°C, liegt.
Mit der Vermischung der beiden Komponenten beginnt die Aushärtung durch chemische Reaktion, wie vorgängig beschrieben. Die Aushärtung erfolgt insbesondere bei Umgebungstemperatur. Sie erstreckt sich typischerweise über einige Tage bis Wochen, bis sie unter den gegebenen Bedingungen weitgehend abgeschlossen ist. Die Dauer hängt unter anderem von der Temperatur, der Reaktivität der Bestandteile und deren Stöchiometrie sowie der Gegenwart von Beschleunigern ab.
Ein weiterer Gegenstand der Erfindung ist somit auch eine ausgehärtete Zusammensetzung erhalten aus der Aushärtung einer Epoxidharz-Zusammensetzung wie im vorliegenden Dokument beschrieben.

Die Applikation der Epoxidharz-Zusammensetzung erfolgt auf mindestens ein Substrat, wobei die Folgenden besonders geeignet sind:
- Glas, Glaskeramik, Beton, Mörtel, Backstein, Ziegel, Gips oder Natursteine wie Granit oder Marmor;
- Metalle oder Legierungen wie Aluminium, Eisen, Stahl oder Buntmetalle, oder oberflächenveredelte Metalle oder Legierungen wie verzinkte oder verchromte Metalle;
- Leder, Textilien, Papier, Holz, mit Harzen, beispielsweise Phenol-, Melamin- oder Epoxidharzen, gebundene Holzwerkstoffe, Harz-Textil-Verbundwerkstoffe oder weitere sogenannte Polymer-Composites;
- Kunststoffe, insbesondere Hart- oder Weich-PVC, ABS, Polycarbonat (PC), Polyamid (PA), Polyester, PMMA, Epoxidharze, PUR, POM, PO, PE, PP, EPM oder EPDM, wobei die Kunststoffe gegebenenfalls mittels Plasma, Corona oder Flammen oberflächenbehandelt sind;
- Faserverstärkte Kunststoffe, wie Kohlefaser-verstärkte Kunststoffe (CFK), Glasfaser-verstärkte Kunststoffe (GFK) oder Sheet Moulding Compounds (SMC);
- beschichtete Substrate, wie pulverbeschichtete Metalle oder Legierungen;
- Farben oder Lacke.

Die Substrate können bei Bedarf vor dem Applizieren der Epoxidharz-Zusammensetzung vorbehandelt werden. Derartige Vorbehandlungen umfassen insbesondere physikalische und/oder chemische Reinigungsverfahren, beispielsweise Schleifen, Sandstrahlen, Kugelstrahlen, Bürsten und/oder Abblasen, sowie weiterhin Behandeln mit Reinigern oder Lösemitteln oder das Aufbringen eines Haftvermittlers, einer Haftvermittlerlösung oder eines Primers.

Die beschriebene Epoxidharz-Zusammensetzung ist vorteilhaft verwendbar als Faserverbundmatrix für Faserverbundwerkstoffe (Composites) wie insbesondere CFK oder GFK, oder als Vergussmasse, Dichtstoff, Klebstoff, Belag, Beschichtung, Anstrich, Lack, Versiegelung, Grundierung oder Primer. Insbesondere verwendbar ist sie als Vergussmasse, beispielsweise als Elektrovergussmasse, oder als Klebstoff, insbesondere als Karrosserieklebstoff, Sandwichelementklebstoff, Halbschalenklebstoff für Rotorblätter von Windkraftanlagen, Brückenelementklebstoff oder Verankerungsklebstoff.
Insbesondere verwendbar ist sie weiterhin als Belag, Beschichtung, Anstrich, Lack, Versiegelung, Grundierung oder Primer für Bau- und Industrieanwendungen, insbesondere als Bodenbelag oder Bodenbeschichtung für Innenräume wie Büros, Industriehallen, Turnhallen oder Kühlräume, oder im Aussenbereich für Balkone, Terrassen, Parkdecks, Brücken oder Dächer, als Schutzbeschichtung für Beton, Zement, Metalle, Kunststoffe oder Holz, beispielsweise zur Oberflächenversiegelung von Holzkonstruktionen, Fahrzeugen, Ladeflächen, Tanks, Silos, Schächten, Rohrleitungen, Pipelines, Maschinen oder Stahlkonstruktionen, beispielsweise von Schiffen, Piers, Offshore-Plattformen, Schleusentoren, Wasserkraftwerken, Flussbauten, Schwimmbädern, Windkraftanlagen, Brücken, Kaminen, Kranen oder Spundwänden.
Insbesondere verwendbar ist sie weiterhin als Voranstrich, Haftanstrich, Korrosionsschutz-Primer oder zur Hydrophobierung von Oberflächen.
Auf die vollständig oder teilweise ausgehärtete Epoxidharz-Zusammensetzung kann insbesondere bei ihrer Verwendung als Beschichtung, Belag oder Anstrich eine weitere Beschichtung, ein weiterer Belag, oder ein weiterer Anstrich appliziert werden, wobei es sich bei dieser weiteren Schicht ebenfalls um eine Epoxidharz-Zusammensetzung handeln kann, aber auch um ein anderes Material, insbesondere um eine Polyurethan- oder Polyharnstoff-Beschichtung.

Besonders vorteilhaft wird die beschriebene Epoxidharz-Zusammensetzung als Beschichtung verwendet.
Ein weiterer Gegenstand der Erfindung ist dementsprechend eine Beschichtung, enthaltend eine Epoxidharz-Zusammensetzung wie vorgängig beschrieben.
Als Beschichtung werden dabei flächig aufgebrachte Beläge aller Art verstanden, insbesondere Anstriche, Lacke, Versiegelungen, Grundierungen oder Primer, wie vorgängig beschrieben, oder Bodenbeläge oder Schutzbeschichtungen, insbesondere auch solche für schweren Korrosionsschutz. Besonders vorteilhaft wird die beschriebene Epoxidharz-Zusammensetzung in emissionsarmen Beschichtungen mit Öko-Gütesiegeln, beispielsweise nach Emicode (EC1 Plus), AgBB, DIBt, Der Blaue Engel, AFSSET, RTS (M1) und US Green Building Council (LEED), verwendet.

Als Beschichtung wird die Epoxidharz-Zusammensetzung vorteilhaft in einem Verfahren zum Beschichten verwendet, wobei sie eine flüssige Konsistenz mit niedriger Viskosität und guten Verlaufseigenschaften aufweist und insbesondere als selbstverlaufende oder thixotropierte Beschichtung auf überwiegend ebene Flächen oder als Anstrich appliziert wird. Bevorzugt weist die Epoxidharz-Zusammensetzung bei dieser Applikation unmittelbar nach dem Vermischen der Harz- und der Härter-Komponente eine Viskosität, gemessen bei 20°C, im Bereich von 300 bis 4'000 mPa·s, bevorzugt im Bereich von 300 bis 2'000 mPa·s, besonders bevorzugt im Bereich von 300 bis 1'500 mPa·s, auf. Die vermischte Zusammensetzung wird innerhalb der Verarbeitungszeit flächig als dünner Film mit einer Schichtdicke von typischerweise etwa 50 µm bis etwa 5 mm auf ein Substrat appliziert, typischerweise bei Umgebungstemperatur. Die Applikation erfolgt insbesondere durch Aufgiessen auf das zu beschichtende Substrat und anschliessendem gleichmässigem Verteilen mit Hilfe beispielsweise eines Rakels oder einer Zahntraufel. Die Applikation kann auch mit einem Pinsel oder Roller oder als Spritzapplikation erfolgen, beispielsweise als Korrosionsschutzbeschichtung auf Stahl.
Bei der Aushärtung entstehen typischerweise weitgehend klare, glänzende und nichtklebrige Filme von hoher Härte, welche eine gute Haftung zu verschiedensten Substraten aufweisen.

Aus der Anwendung der Epoxidharz-Zusammensetzung entsteht ein Artikel umfassend die ausgehärtete Zusammensetzung aus der Aushärtung der beschriebenen Epoxidharz-Zusammensetzung. Die ausgehärtete Zusammensetzung liegt dabei insbesondere in Form einer Beschichtung vor.

Die beschriebene Epoxidharz-Zusammensetzung zeichnet sich durch vorteilhafte Eigenschaften aus. Sie ist sehr niedrigviskos und geruchsarm und härtet auch bei feucht-kaltenBedingungen schnell und weitgehend ohne Blushing-Effekte aus, sogar mit geringen Anteilen oder ganz ohne die Verwendung von nicht einbaubaren Verdünnern, und insbesondere auch ohne die Verwendung von leichtflüchtigen, geruchsintensiven Aminen. Bei der flächigen Verwendung als Beschichtung entstehen klare, nichtklebrige Filme von hoher Härte und hoher Oberflächenqualität, welche unter Lichteinfluss kaum vergilben. Mit der beschriebenen Epoxidharz-Zusammensetzung sind insbesondere emissionsarme Epoxidharz-Produkte zugänglich, welche die Bedingungen für viele Öko-Gütesiegel erfüllen und gleichzeitig hohen Ansprüchen bezüglich Arbeitssicherheit, Verarbeitungs- und Gebrauchseigenschaften genügen.

Ein weiterer Gegenstand der Erfindung ist eine Methode zum Verdünnen eines Härters für Epoxidharze und/oder eines Epoxidharzes, indem ein Amin der Formel (I) wie vorgängig beschrieben zugegeben wird. Der Härter für Epoxidharze enthält dabei insbesondere ein Addukt oder ein Polyamidoamin oder eine Mannich-Base, wie vorgängig beschrieben. Das Epoxidharz stellt dabei ein Epoxidharz wie vorgängig beschrieben dar.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

"AHEW" steht für das Aminwasserstoff-Equivalentgewicht.

"EEW" steht für das Epoxid-Equivalentgewicht.

Als "Normklima" wird eine Temperatur von 23±1 °C und eine relative Luftfeuchtigkeit von 50±5% bezeichnet. "NK" steht für "Normklima".

### Beschreibung der Messmethoden:

**Infrarotspektren** (FT-IR) wurden als unverdünnte Filme auf einem mit horizontaler ATR-Messeinheit mit ZnSe-Kristall ausgestatteten FT-IR Gerät 1600 von Perkin-Elmer gemessen; die Absorptionsbanden sind in Wellenzahlen (cm⁻¹) angegeben (Messfenster: 4000-650 cm⁻¹).

**¹H-NMR-Spektren** wurden auf einem Spektrometer des Typs Bruker Ascend 400 bei 400.14 MHz gemessen; die chemischen Verschiebungen δ sind angegeben in ppm relativ zu Tetramethylsilan (TMS). Echte und Pseudo-Kopplungsmuster wurden nicht unterschieden.

**Gaschromatogramme** (GC) wurden gemessen im Temperaturbereich von 60 bis 320 °C mit einer Aufheizrate von 15 °C/min und 10 min Verweilzeit bei 320 °C. Die Injektortemperatur betrug 250 °C. Es wurde eine Zebron ZB-5 Säule verwendet (L = 30 m, ID = 0.25 mm, dj = 0.5 µm) bei einem Gasfluss von 1.5 ml/Min. Die Detektion erfolgte mittels Flammenionisation (FID).

Die **Viskosität** von höherviskosen Proben (oberhalb von 150 mPa·s) wurde auf einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10 s⁻¹) gemessen.

Die **Viskosität** von niedrigviskosen Proben (unterhalb von 150 mPa·s) wurde auf einem thermostatisierten Kegel-Platten Rheometer Anton Paar Physica MCR 300 (Kegeldurchmesser 25 mm, Kegelwinkel 2°, Kegelspitze-Platten-Abstand 0.05 mm, Scherrate 100 s⁻¹) gemessen.

Die **Aminzahl** wurde durch Titration bestimmt (mit 0.1N HClO₄ in Essigsäure gegen Kristallviolett).

Verwendete Substanzen:

| | |
|---|---|
| Araldite® GY 250: | Bisphenol-A-Diglycidylether, EEW ca. 187.5 g/Eq (von Huntsman) |
| Araldite® DY-E: | Monoglycidylether von C₁₂- bis C₁₄-Alkoholen, EEW ca. 290 g/Eq (von Huntsman) |
| Ancamine® K 54: | 2,4,6-Tris(dimethylaminomethyl)phenol (von Air Products) |
| Araldite® DY-K: | Kresylglycidylether, EEW ca. 182 g/Eq (von Huntsman) |
| EP-Addukt 1: | Umsetzungsprodukt aus 1,5-Diamino-2-methylpentan und Araldite® DY-K, wie nachfolgend beschrieben; AHEW ca. 109.5 g/Eq; Viskosität (20°C) 13'100 mPa·s |
| EP-Addukt 2: | Umsetzungsprodukt aus 1,5-Diamino-2-methylpentan und Araldite® DY-K, wie nachfolgend beschrieben; AHEW ca. 106.5 g/Eq; Viskosität (20°C) 13'000 mPa·s |
| EP-Addukt 3: | Umsetzungsprodukt aus 1,2-Propylendiamin und Araldite® DY-K, wie nachfolgend beschrieben; AHEW ca. 90.0 g/Eq; Viskosität (20°C) 23'000 mPa·s |
| Jeffamine® D-230: | Polyoxypropylendiamin mit mittlerem Molekulargewicht ca. 240 g/mol, AHEW ca. 60 g/Eq (von Huntsman) |
| Gaskamine® 240: | Styrolisiertes 1,3-Bis(aminomethyl)benzol; AHEW 103 g/Eq; Viskosität (20°C) 165 mPa·s (von Mitsubishi Gas Chemical) |
| 1,3-Bis(benzylaminomethyl)benzol: | hergestellt wie N,N'-Dibenzyl-m-xylylendiamin (Amin 1) in WO 2013/010842 |

Das **EP-Addukt 1** wurde hergestellt, indem 116.0 g 1,5-Diamino-2-methylpentan (Dytek® A von Invista) unter Stickstoffatmosphäre vorgelegt, auf 70 °C aufgewärmt und dann unter gutem Rühren langsam mit 200.2 g Araldite® DY-K versetzt wurde, wobei die Temperatur der Reaktionsmischung 70 bis 80 °C betrug. Nach 1 Stunde bei 80°C wurde die Reaktionsmischung abgekühlt.

Das **EP-Addukt 2** wurde hergestellt, indem 4.65 kg 1,5-Diamino-2-methylpentan (Dytek® A von Invista) unter Stickstoffatmosphäre vorgelegt, auf 70 °C aufgewärmt und dann unter gutem Rühren langsam mit 1.83 kg Araldite® DY-K versetzt wurde, wobei die Temperatur der Reaktionsmischung 70 bis 80 °C betrug. Nach 1 Stunde bei 80°C wurde die Reaktionsmischung abgekühlt und die flüchtigen Bestandteile destillativ mittels Dünnschichtverdampfer (0.5-1 mbar, Manteltemperatur 160 °C) entfernt.

Das **EP-Addukt 3** wurde hergestellt, indem 4.15 kg 1,2-Propylendiamin unter Stickstoffatmosphäre vorgelegt, auf 70 °C aufgewärmt und dann unter gutem Rühren langsam mit 2.93 kg Araldite® DY-K versetzt wurde, wobei die Temperatur der Reaktionsmischung 70 bis 80 °C betrug. Nach 1 Stunde bei 80°C wurde die Reaktionsmischung abgekühlt und die flüchtigen Bestandteile destillativ mittels Dünnschichtverdampfer (0.5-1 mbar, Manteltemperatur 115 °C) entfernt.

### Herstellung von Aminen der Formel (I)

### Amin 1: Reaktionsgemisch enthaltend N¹-Benzyl-1,2-propandiamin

In einem Rundkolben wurden 22.1 g (0.3 mol) 1,2-Propandiamin unter Stickstoffatmosphäre bei Raumtemperatur vorgelegt. Unter gutem Rühren wurde langsam eine Lösung von 31.8 g (0.3 mol) Benzaldehyd in 500 ml Isopropanol dazugetropft und 30 min. nachgerührt. Danach wurde die Reaktionsmischung bei einem Wasserstoff-Druck von 85 bar, einer Temperatur von 85 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung wurde im Vakuum bei 65 °C eingeengt. Erhalten wurde eine klare, leicht gelbliche Flüssigkeit mit einer Viskosität von 19 mPa·s bei 20 °C und einer Aminzahl von 573.7 mg KOH/g.
FT-IR: 3026, 2956, 2818, 1601, 1494, 1452, 1373, 1115, 1073, 1028, 826, 732, 696.

### Amin 1A: Reaktionsgemisch enthaltend N¹-Benzyl-1,2-propandiamin

In einem Rundkolben wurden 444.8 g (6 mol) 1,2-Propandiamin unter Stickstoffatmosphäre bei Raumtemperatur vorgelegt. Unter gutem Rühren wurde langsam eine Lösung aus 212.2 g (2 mol) Benzaldehyd in 1'500 ml Isopropanol dazugetropft und 2 Stunden nachgerührt. Die Reaktionsmischung wurde anschliessend bei einem Wasserstoff-Druck von 90 bar, einer Temperatur von 85 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung am Rotationsverdampfer bei 65 °C eingeengt, wobei unreagiertes 1,2-Propandiamin und Isopropanol entfernt wurden. Erhalten wurde eine klare, leicht gelbliche Flüssigkeit mit einer Aminzahl von 574 mg KOH/g.

### Amin 1D: Mischung aus N¹-Benzyl-1,2-propandiamin und N²-Benzyl-1,2-propandiamin

300 g des wie oben hergestellten Amins **1A** wurden bei 80 °C unter Vakuum destilliert, wobei 237.5 g Destillat bei einer Dampftemperatur von 60 bis 63 °C und 0.08 bis 0.09 bar aufgefangen wurden. Erhalten wurde eine farblose Flüssigkeit mit einer Viskosität von 8.5 mPa·s bei 20 °C, einer Aminzahl von 682 mg KOH/g und einer mittels GC bestimmten Reinheit von > 97 % (Retentionszeit 8.73 - 8.83 min). Gemäss ¹H-NMR betrug das Verhältnis zwischen N¹-Benzyl-1,2-propandiamin und N²-Benzyl-1,2-propandiamin ca. 2/1.
¹H-NMR (CDCl₃): 7.23-7.30 (m, 5 H, Ar-H), 3.75 (m, 2 H, Ar-CH₂), 2.9-2.5 (m, 3 H, -CH₂-; C*H*CH₃), 1.32 (b, 3 H, NH und NH₂), 1.03 (t, 3 H, CH₃).
FT-IR: 3361, 3229, 3025, 2956, 2817, 1601, 1494, 1452, 1372, 1115, 1027, 824, 732.

### Amin 2: Reaktionsgemisch enthaltend N,N'-Dibenzyl-1,2-propylendiamin

In einem Rundkolben wurden 7.4 g (0.1 mol) 1,2-Propandiamin unter Stickstoffatmosphäre bei Raumtemperatur vorgelegt. Unter gutem Rühren wurden langsam 17.0 g (0.16 mol) Benzaldehyd dazugetropft, 30 min. nachgerührt und das Reaktionsgemisch in 300 ml Isopropanol gelöst. Die Lösung wurde bei einem Wasserstoff-Druck von 85 bar, einer Temperatur von 85 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine klare, leicht gelbliche Flüssigkeit mit einer Viskosität von 41 mPa·s bei 20 °C und einer Aminzahl von 492.3 mg KOH/g.
FT-IR: 3026, 2959, 2802,1602, 1494, 1452, 1373, 1155, 1115, 1067, 1027, 824, 732, 696.

### Amin 3: Reaktionsgemisch enthaltend N¹-(4-Dimethylaminobenzyl)-1,2-propandiamin

In einem Rundkolben wurden 14.8 g (0.2 mol) 1,2-Propandiamin unter Stickstoffatmosphäre bei Raumtemperatur vorgelegt. Unter gutem Rühren wurde langsam eine Lösung von 29.8 g (0.2 mol) 4-Dimethylaminobenzaldehyd in 450 ml Isopropanol und 50 ml Ethylacetat dazugetropft und 30 min. nachgerührt. Danach wurde das Reaktionsgemisch bei einem Wasserstoff-Druck von 85 bar, einer Temperatur von 85 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine klare, leicht gelbliche Flüssigkeit mit einer Viskosität von 16 mPa·s bei 20 °C und einer Aminzahl von 667.4 mg KOH/g.
FT-IR: 2954, 2917, 2878, 2798, 1614, 1519, 1443, 1340, 1222, 1161, 1130, 1059, 946, 802, 685.

### Amin 3D: Mischung aus N¹-(4-Dimethylaminobenzyl)-1,2-propandiamin und N²-(4-Dimethylaminobenzyl)-1,2-propandiamin

In einem Rundkolben wurden 88.94 g (1.2 mol) 1,2-Propandiamin unter Stickstoffatmosphäre bei Raumtemperatur vorgelegt. Unter gutem Rühren wurde langsam eine Lösung von 44.75 g (0.3 mol) 4-Dimethylaminobenzaldehyd in 800 ml Isopropanol dazugetropft und 2 Stunden nachgerührt. Die Reaktionsmischung wurde bei einem Wasserstoff-Druck von 90 bar, einer Temperatur von 85 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung am Rotationsverdampfer bei 65 °C eingeengt, wobei unreagiertes 1,2-Propylendiamin und Isopropanol entfernt wurden. Die so erhaltene Reaktionsmischung war eine klare, gelbliche Flüssigkeit mit einer Aminzahl von 667 mg KOH/g. 53 g dieser Reaktionsmischung wurden bei 115 °C unter Vakuum destilliert, wobei 41.9 g Destillat bei einer Dampftemperatur von 96 bis 98 °C und 0.02 bar aufgefangen wurden. Erhalten wurde eine farblose Flüssigkeit mit einer Viskosität von 25 mPa·s bei 20 °C, einer Aminzahl von 811.5 mg KOH/g und einer mittels GC bestimmten Reinheit von > 97 % (Retentionszeit 12.21 min und 12.29 min), welche im Folgenden als Amin **3D** eingesetzt wurde. Gemäss GC betrug das Verhältnis zwischen N¹-(4-Dimethylaminobenzyl)-1,2-propandiamin und N²-(4-Dimethylaminobenzyl)-1,2-propandiamin ca. 4/1.
¹H-NMR (CDCl₃): 7.18 (d, 2H, Ar-H), 6.70 (d, 2H, Ar-H), 3.69 (d, 2H, Ar-C*H₂*), 2.96 und 2.65 (2xm, 3 H, -CH₂-; C*H*CH₃), 2.92 (s, 6H, N-(CH₃)₂), 1.33 (b, 3H, NH and NH₂), 1.03 (t, 3 H, CHC*H₃*).
FT-IR: 3299, 2954, 2798, 1613, 1564, 1505, 1442, 1341, 1221, 1161, 1128, 1059, 945, 802.

### Amin 4: Reaktionsgemisch enthaltend N¹-(4-Methoxybenzyl)-1,2-propandiamin

In einem Rundkolben wurden 14.8 g (0.2 mol) 1,2-Propandiamin unter Stickstoffatmosphäre bei Raumtemperatur vorgelegt. Unter gutem Rühren wurde langsam eine Lösung von 27.2 g (0.2 mol) 4-Methoxybenzaldehyd (= Anisaldehyd) in 500 ml Isopropanol dazugetropft und 30 min. nachgerührt. Die Reaktionsmischung wurde bei einem Wasserstoff-Druck von 85 bar, einer Temperatur von 85 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine klare, leicht gelbliche Flüssigkeit mit einer Viskosität von 39 mPa·s bei 20 °C und einer Aminzahl von 501.2 mg KOH/g.
FT-IR: 2955, 2906, 2833, 1610, 1584, 1510,1455, 1441, 1300, 1242, 1173, 1105, 1033, 811, 702.

### Amin 4D: Mischung aus N¹-(4-Methoxybenzyl)-1,2-propandiamin und N²-(4-Methoxybenzyl)-1,2-propandiamin

In einem Rundkolben wurden 148.3 g (2 mol) 1,2-Propandiamin unter Stickstoffatmosphäre bei Raumtemperatur vorgelegt. Unter gutem Rühren wurde langsam eine Lösung von 54.4 g (0.4 mol) 4-Methoxybenzaldehyd (= Anisaldehyd) in 800 ml Isopropanol dazugetropft und 2 Stunden nachgerührt. Die Reaktionsmischung wurde bei einem Wasserstoff-Druck von 90 bar, einer Temperatur von 90 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung am Rotationsverdampfer bei 65 °C eingeengt, wobei unreagiertes 1,2-Propylendiamin und Isopropanol entfernt wurden. Die so erhaltene Reaktionsmischung war eine klare, leicht gelbliche Flüssigkeit mit einer Aminzahl von 560 mg KOH/g. 40 g dieser Reaktionsmischung wurden bei 110 °C unter Vakuum destilliert, wobei 29.6 g Destillat bei einer Dampftemperatur von 89 bis 95 °C und 0.02 bar aufgefangen wurden. Erhalten wurde eine farblose Flüssigkeit mit einer Viskosität von 23 mPa·s bei 20 °C, einer Aminzahl von 575 mg KOH/g und einer mittels GC bestimmten Reinheit von > 97 % (Retentionszeit 10.85 - 10.95 min), welche im Folgenden als Amin **4D** eingesetzt wurde.
¹H-NMR (CDCl₃): 7.22 (d, 2 H, Ar-H), 6.85 (d, 2 H, Ar-H), 3.78 (s, 3 H, OCH*₃*), 3.71 (d, 2 H, Ar-C*H*₂), 2.9- 2.5 (m, 3 H, -CH₂-; C*H*CH₃), 1.32 (b, 3 H, NH und NH₂), 1.04 (t, 3 H, CHC*H₃*).
FT-IR: 3367, 3298, 2955, 2924, 2832, 1610, 1584, 1509, 1461, 1299, 1248, 1173, 1033, 811.

### Amin 5: Reaktionsgemisch enthaltend N-Benzyl-1,3-propandiamin (Vergleich)

In einem Rundkolben wurden 148.3 g (2 mol) 1,3-Propandiamin unter Stickstoffatmosphäre bei Raumtemperatur vorgelegt. Unter gutem Rühren wurde langsam eine Lösung aus 42.4 g (0.4 mol) Benzaldehyd in 800 ml Isopropanol dazugetropft und 2 Stunden nachgerührt. Die Reaktionsmischung wurde anschliessend bei einem Wasserstoff-Druck von 90 bar, einer Temperatur von 90 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung am Rotationsverdampfer bei 65 °C eingeengt, wobei unreagiertes 1,3-Propandiamin und Isopropanol entfernt wurden. Erhalten wurde eine klare, leicht gelbliche Flüssigkeit mit einer Aminzahl von 569 mg KOH/g.

### Amin 5D: N-Benzyl-1,3-propandiamin (Vergleich)

30 g des wie oben hergestellten Amins **5** wurden bei 90 °C unter Vakuum destilliert, wobei 20.3 g Destillat bei einer Dampftemperatur von 68 bis 73 °C und 0.06 bar aufgefangen wurden. Erhalten wurde eine farblose Flüssigkeit mit einer Viskosität von 10.8 mPa·s bei 20 °C, einer Aminzahl von 682 mg KOH/g und einer mittels GC bestimmten Reinheit von > 97 % (Retentionszeit 9.39 - 9.46 min).

### Blushing der hergestellten Amine

Von jedem Amin gemäss Tabelle 1 wurde 1 g in eine offene Schale mit einem Durchmesser von 4 cm gegeben und im Normklima aufbewahrt. Bei jeder Probe lag das Amin zu Beginn als klare Flüssigkeit vor. Nach 16 und nach 48 Stunden wurde jeweils der Aspekt beurteilt. Zusätzlich wurde nach 72 Stunden die Gewichtszunahme der Probe bestimmt.

**Tabelle 1: Blushing von offen gelagerten Aminen im NK.**

| **Probe** | optische Beurteilung | | Gewichtszunahme nach 72 h |
|---|---|---|---|
| | nach 16 h | nach 48 h | |
| Amin 1D¹ | klare Flüssigkeit | klare Flüssigkeit | 0.22 g |
| Amin 4D¹ | klare Flüssigkeit | klare Flüssigkeit | 0.19 g |
| Amin 5D¹ | weisse Flecken auf der Oberfläche | weisse Kruste auf der Oberfläche | 0.29 g |
| IPDA² | weisse Flecken auf der Oberfläche | weisse Kruste auf der Oberfläche | 0.33 g |
| MXDA³ | weiss-trübe Oberfläche | weisse Kruste auf der Oberfläche | 0.33 g |
| D230⁴ | klare Flüssigkeit | klare Flüssigkeit | 0.24 g |

| | | | |
|---|---|---|---|
| ¹ wie vorgängig hergestellt ² 1-Amino-3-aminomethyl-3,5,5-trimethylcyclohexan ³ 1,3-Bis(aminomethyl)benzol ⁴ Jeffamine® D-230 | | | |

### Herstellung von Härtern und Epoxidharz-Zusammensetzungen

Für jedes Beispiel wurden die in den Tabellen 2 bis 6 angegebenen Inhaltsstoffe in den angegebenen Mengen (in Gewichtsteilen) der Härter-Komponente mittels eines Zentrifugalmischers (SpeedMixer™ DAC 150, FlackTek Inc.) vermischt und unter Ausschluss von Feuchtigkeit aufbewahrt.

Ebenso wurden die in den Tabellen 2 bis 6 angegebenen Inhaltsstoffe der Harz-Komponente verarbeitet und aufbewahrt.

Anschliessend wurden die beiden Komponenten jeder Zusammensetzung mittels des Zentrifugalmischers zu einer homogenen Flüssigkeit verarbeitet und diese unverzüglich folgendermassen geprüft:
10 Minuten nach dem Vermischen wurde die Viskosität bei 20°C bestimmt ("**Viskosität (10')**").

Ein erster Film wurde in einer Schichtdicke von 500 µm auf eine Glasplatte aufgezogen und dieser Normklima gelagert bzw. ausgehärtet. An diesem Film wurde die **Königshärte** (Pendelhärte nach König, gemessen nach DIN EN ISO 1522) nach 1 Tag ("Königshärte (1d NK)"), nach 2 Tagen ("Königshärte (2d NK)"), nach 4 Tagen ("Königshärte (4d NK)"), nach 7 Tagen ("Königshärte (7d NK)") und nach 14 Tagen ("Königshärte (14d NK)") bestimmt. Nach 14 Tagen wurde der Aspekt des Films beurteilt (in der Tabelle mit "Aspekt (NK)" bezeichnet). Als "schön" wurde dabei ein Film bezeichnet, welcher klar war und eine glänzende und nichtklebrige Oberfläche ohne Struktur aufwies. Als "Struktur" wird dabei jegliche Art von Zeichnung oder Muster auf der Oberfläche bezeichnet.

Ein zweiter Film wurde in einer Schichtdicke von 500 µm auf eine Glasplatte aufgezogen und dieser unmittelbar nach dem Applizieren während 7 Tagen bei 8 °C und 80 % relativer Feuchtigkeit und anschliessend während 3 Wochen im NK gelagert, beziehungsweise ausgehärtet. 24 Stunden nach der Applikation wurde ein Flaschendeckel aus Polypropylen auf den Film aufgesetzt, unter welchem ein feuchtes Schwämmchen platziert war. Nach weiteren 24 Stunden wurde das Schwämmchen und der Deckel entfernt und an einer neuen Stelle des Films platziert, wo es nach 24 Stunden wieder entfernt und neu platziert wurde, insgesamt 4 mal. Anschliessend wurde der Aspekt dieses Films beurteilt (in den Tabellen mit "Aspekt (8°/80%)" bezeichnet), auf die gleiche Weise wie für den Aspekt (NK) beschrieben. Dabei wurde jeweils auch die Anzahl Markierungen angegeben, die im Film durch das feuchte Schwämmchen und/ oder den aufgesetzten Deckel sichtbar waren. An den so ausgehärteten Filmen wurde wiederum die Königshärte bestimmt, jeweils nach 7 Tagen bei 8 °C und 80 % relativer Feuchtigkeit ("Königsh. (7d 8°/80%)"), dann nach weiteren 2 Tagen im NK ("Königsh. (+2d NK)"), 7 Tagen im NK ("Königsh. (+7d NK)") und nach 14d im NK ("Königsh. (+14d NK)") oder nach 3 Wochen im NK ("Königsh. (+3w NK)").

Die **Vergilbung** wurde einerseits bestimmt, indem ein weiterer Film in einer Schichtdicke von 500 µm auf eine Glasplatte aufgezogen und dieser im Normklima während 4 Wochen gelagert bzw. ausgehärtet, anschliessend eine Hälfte des Films mit Alufolie abgedeckt und der Film im Labor dem Tageslicht ausgesetzt wurde. Nach 3 Monaten wurde die Alufolie entfernt und die Vergilbung aufgrund des beobachteten Farbunterschieds zwischen der abgedeckten und der freien Zone des Films von Auge beurteilt. Dabei steht "keine" für keinen erkennbaren Farbunterschied, "leicht" für einen kleinen Farbunterschied, "mittel" für einen deulichen Farbunterschied und "stark" für einen starken Farbunterschied.

Als Mass für die Vergilbung wurde weiterhin die Farbveränderung nach Belastung in einem Bewitterungstester bestimmt. Dazu wurde ein weiterer Film in einer Schichtdicke von 500 µm auf eine Glasplatte aufgezogen und dieser im Normklima während 2 Wochen gelagert bzw. ausgehärtet und anschliessend in einem Bewitterungstester des Typs Q-Sun Xenon Xe-1 mit optischem Filter Q-SUN Daylight-Q und einer Xenon Lampe mit einer Lichtstärke von 0.51 W/m² bei 340 nm bei einer Temperatur von 65°C während 72 Stunden belastet **(Q-Sun (72h)).** Anschliessend wurde der Farbunterschied ΔE des so belasteten Films im Vergleich zum entsprechenden nicht belasteten Film mittels einem Colorimeter NH310 von Shenzen 3NH Technology Co. LTD, ausgerüstet mit Silicon Photoelectric Diode Detector, Light Source A, Color Space Measurement Interface CIE L*a*b*C*H*, bestimmt. ΔE Werte von 0.5 bis 1.5 stehen dabei für einen geringfügigen Farbunterschied, 1.5 bis 3 für einen merklichen Farbunterschied, 3 bis 6 für einen deutlich sichtbaren Farbunterschied und mehr als 6 für einen grossen Farbunterschied.

Die Resultate sind in den Tabellen 2 bis 6 angegeben.

Bei den Epoxidharz-Zusammensetzungen ***Ex-1*** bis ***Ex-18*** handelt es sich um erfindungsgemässe Beispiele. Bei den Epoxidharz-Zusammensetzungen ***Ref-1*** bis ***Ref-10*** handelt es sich um Vergleichsbeispiele.

**Tabelle 2: Zusammensetzung und Eigenschaften von Ex-1 bis Ex-3 und Ref-1 bis Ref-2. "n.b." steht für "nicht bestimmt"**

| **Beispiel** | | | | ***Ex-1*** | ***Ex-2*** | ***Ex-3*** | ***Ref-1*** | ***Ref-2*** |
|---|---|---|---|---|---|---|---|---|
| **Harz-Komp.:** | | | | | | | | |
| | Araldite® GY-250 | | | 167.2 | 167.2 | 167.2 | 167.2 | 167.2 |
| | Araldite® DY-E | | | 31.8 | 31.8 | 31.8 | 31.8 | 31.8 |

| **Härter-Komp.:** | | | | | | | | |
|---|---|---|---|---|---|---|---|---|
| | Amin | | | **1** | **3** | **4** | - | - |
| | | | | 54.8 | 69.1 | 64.8 | | |
| | Gaskamine® 240 | | | - | - | - | 103.0 | - |
| 1,3-Bis(benzylaminomethyl)benzol | | | | - | - | - | - | 158.2 |
| Ancamine® K 54 | | | | 5.1 | 5.4 | 5.3 | 6.0 | 7.1 |
| Viskosität (10') [Pa·s] | | | | 0.48 | 0.47 | 0.60 | 0.68 | 0.34 |
| Königshärte [s] | | (1d NK) | | 41 | 11 | 39 | 70 | n.b. |
| | | (2d NK) | | 111 | 50 | 112 | 127 | 27 |
| | | (4d NK) | | 165 | 112 | 168 | 167 | 108 |
| | | (7d NK) | | 185 | 147 | 197 | 179 | 146 |
| | | (14d NK) | | 210 | 182 | 217 | 195 | 154 |
| Aspekt (NK) | | | | schön | schön | schön | schön | schön |
| Vergilbung | | | | leicht | leicht | leicht | stark | stark |
| Königsh. [s] | | (7d 8°/80%) | | 36 | 31 | 46 | 83 | 6 |
| | | (+2d NK) | | 151 | 119 | 147 | 133 | 78 |
| | | (+7d NK) | | 190 | 167 | 196 | 169 | 161 |
| | | (+3w NK) | | 214 | 202 | 209 | 174 | 169 |
| Aspekt (8°/80%) | | | | schön | schön | schön | schön | schön |
| Anzahl Markierungen | | | | 1 | 1 | 1 | 1 | 3 |

**Tabelle 3: Zusammensetzung und Eigenschaften von Ex-4 bis Ex-7 und Ref-4 bis Ref-6. "n.b." steht für "nicht bestimmt" "n.m." steht für "nicht messbar" aufgrund der trüben Oberfläche**

| **Beispiel** | | | | ***Ex-4*** | ***Ex-5*** | ***Ex-6*** | ***Ex-7*** | ***Ref-4*** | ***Ref-5*** | ***Ref-6*** |
|---|---|---|---|---|---|---|---|---|---|---|
| **Harz-Komp.:** | | | | | | | | | | |
| | Araldite® GY-250 | | | 167.2 | 167.2 | 167.2 | 167.2 | 167.2 | 167.2 | 167.2 |
| | Araldite® DY-E | | | 31.8 | 31.8 | 31.8 | 31.8 | 31.8 | 31.8 | 31.8 |

| **Härter-Komp.:** | | | | | | | | | | |
|---|---|---|---|---|---|---|---|---|---|---|
| | Amin | | | **1A** | **1D** | **3D** | **4D** | **5** | **5D** | - |
| | | | | 54.7 | 54.7 | 69.1 | 64.8 | 54.7 | 54.7 | |
| | Gaskamine® 240 | | | - | - | - | - | - | - | 103.0 |
| Viskosität (10') [Pa·s] | | | | 0.34 | 0.30 | 0.47 | 0.43 | 0.57 | 0.36 | 0.60 |
| Königshärte [s] | | (1d NK) | | 21 | 61 | 104 | 73 | 14 | 38 | 29 |
| | | (2d NK) | | 87 | 137 | 172 | 153 | 25 | 53 | 77 |
| | | (4d NK) | | 141 | 185 | 203 | 186 | 50 | 70 | 113 |
| | | (7d NK) | | 165 | 195 | 209 | 195 | 73 | 85 | 132 |
| | | (14d NK) | | 186 | 209 | 209 | 207 | 127 | 101 | 143 |
| Aspekt (NK) | | | | schön | schön | schön | schön | Flecken | trüb | schön |
| Q-Sun (72h) ΔE | | | | n.b. | 1.1 | n.b. | 3.5 | n.b. | 13.2 | 17.3 |
| Königsh. [s] | | (7d 8°/80%) | | 25 | 70 | 119 | 63 | 8 | 27 | 36 |
| | | (+2d NK) | | 84 | 164 | 199 | 154 | 28 | 63 | 85 |
| | | (+7d NK) | | 116 | 189 | 214 | 160 | 78 | 106 | 116 |
| | | (+14d NK) | | 144 | 204 | 217 | 171 | 102 | 109 | 126 |
| Aspekt (8°/80%) | | | | schön | schön | schön | schön | trüb | trüb | schön |
| Anzahl Markierungen | | | | 1 | 1 | 3 | 1 | n.m. | 2 | 1 |

**Tabelle 4: Zusammensetzung und Eigenschaften von Ex-8 bis Ex-11 und Ref-3. "I." steht für "leicht"**

| **Beispiel** | | | | ***Ex-8*** | ***Ex-9*** | ***Ex-10*** | ***Ex-11*** | ***Ref-3*** |
|---|---|---|---|---|---|---|---|---|
| **Harz-Komp.:** | | | | | | | | |
| | Araldite® GY-250 | | | 167.2 | 167.2 | 167.2 | 167.2 | 167.2 |
| | Araldite® DY-E | | | 31.8 | 31.8 | 31.8 | 31.8 | 31.8 |

| **Härter-Komp.:** | | | | | | | | |
|---|---|---|---|---|---|---|---|---|
| | EP-Addukt-1 | | | 65.7 | 65.7 | 65.7 | 65.7 | 65.7 |
| | Amin | | | **1** | **2** | **3** | **4** | - |
| | | | | 21.9 | 35.9 | 27.6 | 25.9 | |
| | Gaskamine® 240 | | | - | - | - | - | 41.2 |
| | Ancamine® K 54 | | | 5.7 | 6.0 | 5.9 | 5.8 | 6.1 |
| Viskosität (10') [Pa·s] | | | | 2.17 | 2.05 | 2.13 | 2.22 | 2.42 |
| Königshärte [s] | | (1d NK) | | 48 | 25 | 56 | 83 | 102 |
| | | (2d NK) | | 148 | 119 | 113 | 144 | 144 |
| | | (4d NK) | | 171 | 150 | 148 | 174 | 164 |
| | | (7d NK) | | 183 | 165 | 167 | 188 | 174 |
| | | (14d NK) | | 200 | 179 | 179 | 203 | 192 |
| Aspekt (NK) | | | | l. matt | l. matt | l. matt | l. matt | l. matt |
| Königsh. [s] | | (7d 8°/80%) | | 57 | 48 | 63 | 73 | 59 |
| | | (+2d NK) | | 144 | 113 | 136 | 154 | 136 |
| | | (+7d NK) | | 183 | 147 | 168 | 186 | 161 |
| | | (+3w NK) | | 196 | 169 | 197 | 209 | 174 |
| Aspekt (8°/80%) | | | | l. matt | l. matt | l. matt | l. matt | l. matt |
| Anzahl Markierungen | | | | 1 | 1 | 1 | 1 | 1 |

**Tabelle 5: Zusammensetzung und Eigenschaften von Ex-13 bis Ex-16 und Ref-7 bis Ref-8.**

| **Beispiel** | | | | ***Ex-13*** | ***Ex-14*** | ***Ex-15*** | ***Ex-16*** | ***Ref-7*** | ***Ref-8*** |
|---|---|---|---|---|---|---|---|---|---|
| **Harz-Komp.:** | | | | | | | | | |
| | Araldite® GY-250 | | | 167.2 | 167.2 | 167.2 | 167.2 | 167.2 | 167.2 |
| | Araldite® DY-E | | | 31.8 | 31.8 | 31.8 | 31.8 | 31.8 | 31.8 |

| **Härter-Komp.:** | | | | | | | | | |
|---|---|---|---|---|---|---|---|---|---|
| | EP-Addukt-2 | | | - | 42.6 | 42.6 | - | 42.6 | - |
| | EP-Addukt-3 | | | 36.0 | - | - | - | - | - |
| | Jeffamine® D-230 | | | 24.0 | 24.0 | 24.0 | 12.0 | 24.0 | 12.0 |
| | Amin | | | **1D** | **1D** | **1D** | **1D** | - | - |
| | | | | 11.0 | 11.0 | 11.0 | 43.8 | | |
| | Gaskamine® 240 | | | - | - | - | - | 20.6 | 82.4 |
| | Salicylsäure | | | - | - | 1.6 | - | - | - |
| | Ancamine® K 54 | | | - | - | 1.6 | - | - | - |
| Viskosität (10') [Pa·s] | | | | 0.82 | 0.82 | 1.13 | 0.26 | 0.96 | 0.60 |
| Königshärte [s] | | (1d NK) | | 35 | 38 | 55 | 38 | 31 | 15 |
| | | (2d NK) | | 109 | 76 | 129 | 129 | 78 | 61 |
| | | (4d NK) | | 161 | 148 | 163 | 176 | 126 | 104 |
| | | (7d NK) | | 179 | 168 | 177 | 195 | 151 | 130 |
| | | (14d NK) | | 193 | 184 | 191 | 209 | 167 | 139 |
| Aspekt (NK) | | | | schön | schön | schön | schön | schön | schön |
| Q-Sun (72h) ΔE | | | | 1.4 | 2.8 | 10.4 | 1.7 | 7.2 | 14.8 |
| Königsh. [s] | | (7d 8°/80%) | | 40 | 26 | 44 | 27 | 28 | 26 |
| | | (+2d NK) | | 122 | 90 | 137 | 76 | 97 | 77 |
| | | (+7d NK) | | 161 | 102 | 153 | 105 | 136 | 116 |
| | | (+14d NK) | | 168 | 119 | 156 | 108 | 144 | 127 |
| Aspekt (8°/80%) | | | | schön | schön | schön | schön | schön | schön |
| Anzahl Markierungen | | | | 1 | 1 | keine | 1 | 1 | 1 |

**Tabelle 6: Zusammensetzung und Eigenschaften von Ex-17 bis Ex-18 und Ref-9 bis Ref-10..**

| **Beispiel** | | | | ***Ex-17*** | ***Ex-18*** | ***Ref-9*** | ***Ref-10*** |
|---|---|---|---|---|---|---|---|
| **Harz-Komp.:** | | | | | | | |
| | Araldite® GY-250 | | | 167.2 | 167.2 | 167.2 | 167.2 |
| | Araldite® DY-E | | | 31.8 | 31.8 | 31.8 | 31.8 |

| **Härter-Komp.:** | | | | | | | |
|---|---|---|---|---|---|---|---|
| | EP-Addukt-2 | | | 53.3 | - | - | - |
| | EP-Addukt-3 | | | | 45.0 | 45.0 | 45.0 |
| | Amin | | | **1D** | **1D** | **5D** | - |
| | | | | 27.4 | 27.4 | 27.4 | |
| | Gaskamine® 240 | | | - | - | - | 51.5 |
| Viskosität (10') [Pa·s] | | | | 1.35 | 1.28 | 1.39 | 1.66 |
| Königshärte [s] | | (1d NK) | | 78 | 67 | 62 | 43 |
| | | (2d NK) | | 133 | 143 | 109 | 87 |
| | | (4d NK) | | 168 | 180 | 147 | 134 |
| | | (7d NK) | | 182 | 192 | 171 | 148 |
| | | (14d NK) | | 196 | 206 | 187 | 175 |
| Aspekt (NK) | | | | schön | schön | schön | schön |
| Q-Sun (72h) ΔE | | | | 3.2 | 1.7 | 3.7 | 5.0 |
| Königsh. [s] | | (7d 8°/80%) | | 55 | 60 | 43 | 14 |
| | | (+2d NK) | | 158 | 170 | 132 | 119 |
| | | (+7d NK) | | 182 | 192 | 173 | 157 |
| | | (+14d NK) | | 189 | 197 | 186 | 176 |
| Aspekt (8°/80%) | | | | schön | schön | schön | schön |
| Anzahl Markierungen | | | | 1 | 1 | 2 | 1 |

## Patentansprüche

1. Verwendung eines Amins der Formel (I) als Härter für Epoxidharze, wobei 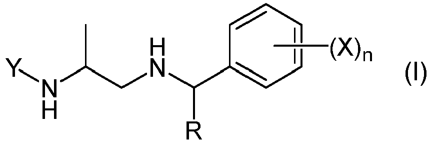
n für 0 oder 1 oder 2 oder 3 steht,
R für einen Wasserstoff-Rest oder für Methyl oder Phenyl steht,
X für gleiche oder verschiedene Reste ausgewählt aus der Gruppe bestehend aus Alkyl, Alkoxy und Dialkylamino mit jeweils1 bis 18 Kohlenstoffatomen steht, und
Y für einen Wasserstoff-Rest oder einen Rest der Formel 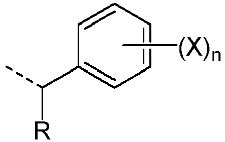 steht.

2. Verwendung gemäss Anspruch 1, **dadurch gekennzeichnet, dass** R für einen Wasserstoff-Rest oder für Methyl steht.

3. Verwendung gemäss einem der Ansprüche 1 oder 2, **dadurch gekennzeichnet, dass** Y für einen Wasserstoff-Rest und n für 0 steht.

4. Verwendung gemäss einem der Ansprüche 1 bis 3, **dadurch gekennzeichnet, dass** Y und R jeweils für einen Wasserstoff-Rest und n für 0 stehen.

5. Verwendung gemäss einem der Ansprüche 1 oder 2, **dadurch gekennzeichnet, dass** Y für einen Wasserstoff-Rest, n für 1 und X für Methoxy oder für Dimethylamino steht.

6. Verwendung eines Reaktionsprodukts aus der reduktiven Alkylierung von 1,2-Propylendiamin mit mindestens einem Aldehyd oder Keton der Formel (II) und Wasserstoff, enthaltend mindestens ein Amin der Formel (I) wie in einem der Ansprüche 1 bis 5 beschrieben, als Härter für Epoxidharze, 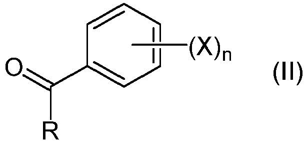
wobei n für 0 oder 1 oder 2 oder 3 steht,
R für einen Wasserstoff-Rest oder für Methyl oder Phenyl steht, und
X für gleiche oder verschiedene Reste ausgewählt aus der Gruppe bestehend aus Alkyl, Alkoxy und Dialkylamino mit jeweils 1 bis 18 Kohlenstoffatomen steht.

7. Härter für Epoxidharze enthaltend mindestens ein Amin der Formel (I) wie in einem der Ansprüche 1 bis 5 beschrieben und mindestens ein weiteres Amin und/oder mindestens einen Beschleuniger.

8. Härter gemäss Anspruch 7, **dadurch gekennzeichnet, dass** der Beschleuniger Salicylsäure oder 2,4,6-Tris(dimethylaminomethyl)phenol oder eine Kombination davon ist.

9. Härter gemäss einem der Ansprüche 7 oder 8, **dadurch gekennzeichnet, dass** das weitere Amin mindestens ein Addukt aus (i) mindestens einem Polyamin mit mindestens drei gegenüber Epoxidgruppen reaktive Aminwasserstoffen mit (ii) mindestens einem Epoxid umfasst.

10. Härter gemäss einem der Ansprüche 7 bis 9, **dadurch gekennzeichnet, dass** 1 bis 95 Gewichts-% Amin der Formel (I) enthalten ist.

11. Härter gemäss einem der Ansprüche 7 bis 10, **dadurch gekennzeichnet, dass** er maximal 5 Gewichts-% nicht einbaubare Verdünner enthält.

12. Epoxidharz-Zusammensetzung umfassend
- eine Harz-Komponente enthaltend mindestens ein Epoxidharz und
- eine Härter-Komponente enthaltend mindestens ein Amin der Formel (I) wie in einem der Ansprüche 1 bis 5 beschrieben.

13. Beschichtung enthaltend eine Epoxidharz-Zusammensetzung wie in Anspruch 12 beschrieben.

14. Ausgehärtete Zusammensetzung erhalten aus der Aushärtung einer Epoxidharz-Zusammensetzung gemäss einem der Ansprüche 12 oder 13.

15. Methode zum Verdünnen eines Härters für Epoxidharze und/oder eines Epoxidharzes, **dadurch gekennzeichnet, dass** ein Amin der Formel (I) wie in einem der Ansprüche 1 bis 4 beschrieben zugegeben wird.

## Claims

1. Use of an amine of the formula (I) as hardener for epoxy resins, 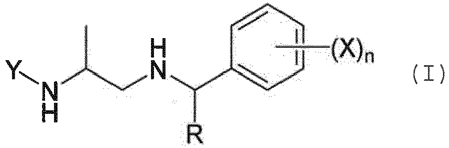 where
n is 0 or 1 or 2 or 3,
R is a hydrogen radical or is methyl or phenyl,
X is identical or different radicals selected from the group consisting of alkyl, alkoxy and dialkylamino having in each case 1 to 18 carbon atoms, and
Y is a hydrogen radical or a radical of the formula

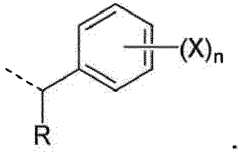

2. The use as claimed in claim 1, **characterized in that** R is a hydrogen radical or is methyl.

3. The use as claimed in either of claims 1 and 2, **characterized in that** Y is a hydrogen radical and n is 0.

4. The use as claimed in any of claims 1 to 3, **characterized in that** Y and R are each a hydrogen radical and n is 0.

5. The use as claimed in either of claims 1 and 2, **characterized in that** Y is a hydrogen radical, n is 1 and X is methoxy or is dimethylamino.

6. Use of a reaction product from the reductive alkylation of 1,2-propylenediamine with at least one aldehyde or ketone of the formula (II) and hydrogen, comprising at least one amine of the formula (I) as described in any of claims 1 to 5 as hardener for epoxy resins.

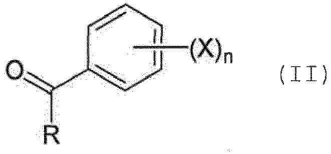

7. A hardener for epoxy resins, comprising at least one amine of the formula (I) as described in any of claims 1 to 5 and at least one further amine and/or at least one accelerator.

8. The hardener as claimed in claim 7, **characterized in that** the accelerator is salicylic acid or 2,4,6-tris(dimethylaminomethyl)phenol or a combination thereof.

9. The hardener as claimed in either of claims 7 and 8, **characterized in that** the further amine comprises at least one adduct of (i) at least one polyamine, having at least three amine hydrogens reactive toward epoxide groups, with (ii) at least one epoxide.

10. The hardener as claimed in any of claims 7 to 9, **characterized in that** 1 to 95 weight% of amine of the formula (I) is present.

11. The hardener as claimed in any of claims 7 to 10, **characterized in that** it contains not more than 5 weight% of unincorporable diluents.

12. An epoxy resin composition comprising
- a resin component comprising at least one epoxy resin and
- a hardener component comprising at least one amine of the formula (I) as described in any of claims 1 to 5.

13. A coating comprising an epoxy resin composition as described in claim 12.

14. A cured composition obtained from the curing of an epoxy resin composition as claimed in either of claims 12 and 13.

15. A method for the dilution of a hardener for epoxy resins and/or of an epoxy resin, **characterized in that** an amine of the formula (I) as described in any of claims 1 to 4 is added.

## Revendications

1. Utilisation d'une amine de formule (I) en tant que durcisseur pour résines époxydes 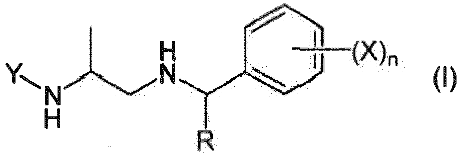 dans laquelle
n représente 0 ou 1 ou 2 ou 3,
R représente un radical hydrogène ou méthyle ou phényle,
X représente des radicaux identiques ou différents choisis dans le groupe constitué par alkyle, alcoxy et dialkylamino contenant chacun 1 à 18 atomes de carbone, et
Y représente un radical hydrogène ou un radical de formule

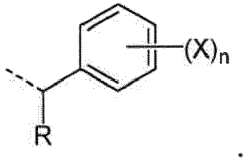

2. Utilisation selon la revendication 1, **caractérisée en ce que** R représente un radical hydrogène ou méthyle.

3. Utilisation selon l'une quelconque des revendications 1 ou 2, **caractérisée en ce qu'**Y représente un radical hydrogène et n représente 0.

4. Utilisation selon l'une quelconque des revendications 1 à 3, **caractérisée en ce qu'**Y et R représentent chacun un radical hydrogène, et n représente 0.

5. Utilisation selon l'une quelconque des revendications 1 ou 2, **caractérisée en ce qu'**Y représente un radical hydrogène, n représente 1 et X représente méthoxy ou diméthylamino.

6. Utilisation d'un produit de réaction de l'alkylation réductrice de 1,2-propylène-diamine avec au moins un aldéhyde ou une cétone de formule (II) et de l'hydrogène, contenant au moins une amine de formule (I) telle que décrite dans l'une quelconque des revendications 1 à 5, en tant que durcisseur pour résines époxydes 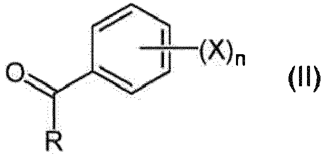
dans laquelle n représente 0 ou 1 ou 2 ou 3,
R représente un radical hydrogène ou méthyle ou phényle,
X représente des radicaux identiques ou différents choisis dans le groupe constitué par alkyle, alcoxy et dialkylamino contenant chacun 1 à 18 atomes de carbone.

7. Durcisseur pour résines époxydes contenant au moins une amine de formule (I) telle que décrite dans l'une quelconque des revendications 1 à 5 et au moins une autre amine et/ou au moins un accélérateur.

8. Durcisseur selon la revendication 7, **caractérisé en ce que** l'accélérateur est l'acide salicylique ou le 2,4,6-tris(diméthylaminométhyl)phénol ou une combinaison de ceux-ci.

9. Durcisseur selon l'une quelconque des revendications 7 ou 8, **caractérisé en ce que** l'autre amine comprend au moins un adduit de (i) au moins une polyamine contenant au moins trois hydrogènes d'amine réactifs avec les groupes époxyde avec (ii) au moins un époxyde.

10. Durcisseur selon l'une quelconque des revendications 7 à 9, **caractérisé en ce que** 1 à 95 % en poids d'amine de formule (I) est contenue.

11. Durcisseur selon l'une quelconque des revendications 7 à 10, **caractérisé en ce qu'**il contient au plus 5 % en poids de diluants non incorporables.

12. Composition de résine époxyde, comprenant :
- une composant résine contenant au moins une résine époxyde et
- un composant durcisseur contenant au moins une amine de formule (I) telle que décrite dans l'une quelconque des revendications 1 à 5.

13. Revêtement contenant une composition de résine époxyde telle que décrite dans la revendication 12.

14. Composition durcie obtenue par le durcissement d'une composition de résine époxyde selon l'une quelconque des revendications 12 ou 13.

15. Méthode de dilution d'un durcisseur pour résines époxydes et/ou d'une résine époxyde, **caractérisée en ce qu'**une amine de formule (I) telle que décrite dans l'une quelconque des revendications 1 à 4 est ajoutée.
